(19) Europäisches Patentamt
European Patent Office
Office européen des brevets

(11) EP 2 826 806 A1

(12) # EUROPÄISCHE PATENTANMELDUNG

(43) Veröffentlichungstag:
**21.01.2015 Patentblatt 2015/04**

(21) Anmeldenummer: **14172890.7**

(22) Anmeldetag: **18.06.2014**

(51) Int Cl.:
*C08G 77/388* (2006.01) *C07C 227/18* (2006.01)
*C07C 279/14* (2006.01) *C07C 211/63* (2006.01)
*C07C 229/26* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **18.07.2013 DE 102013214081**

(71) Anmelder: **Evonik Industries AG**
**45128 Essen (DE)**

(72) Erfinder:
- **Knott, Wilfried**
  **45355 Essen (DE)**
- **Henning, Frauke**
  **45259 Essen (DE)**
- **Amajjahe, Sadik**
  **40591 Düsseldorf (DE)**
- **Krauskopf, Sarah**
  **44867 Bochum (DE)**
- **Fiedel, Michael**
  **45131 Essen (DE)**
- **Jazkewitsch, Olga**
  **45131 Essen (DE)**
- **Hartung, Christian**
  **45133 Essen (DE)**

(54) **Neue aminosäuremodifizierte Siloxane, Verfahren zu ihrer Herstellung und Anwendung**

(57) Gegenstand der Erfindung sind neue aminosäuremodifizierte Siloxane, ihre Herstellverfahren und ihre Anwendung in Pflegeformulierungen für Haut, Haar und Textilien.

EP 2 826 806 A1

## Beschreibung

Gebiet der Erfindung

Die Erfindung betrifft neue aminosäuremodifizierte Siloxane, ihre Herstellverfahren und ihre Anwendung in Pflegeformulierungen für Haut, Haar und Textilien.

Stand der Technik

Stickstoff-Funktionalität enthaltende, insbesondere Aminogruppen tragende Siloxane beanspruchen zunehmende Bedeutung im Bereich der Textilausrüstung, aber auch für wichtige leave-on-Anwendungen im Kosmetikadditivbereich wie z.B. der Haarkonditionierung. Nicht zuletzt unter Aspekten der Nachhaltigkeit, aber auch der Biomimetik sind hierbei diejenigen Stoffsysteme interessant, die auf natürliche Edukte wie Aminosäuren, Proteine und deren Derivate zurückgreifen.
Die chemische Verknüpfung der von diametraler stofflicher Andersartigkeit gekennzeichneten Siloxan- und Aminosäure- bzw. Proteinstrukturen stellt stets eine synthetische Herausforderung dar. So gilt es, die auf dem unterschiedlichen Löslichkeitsverhalten von Siloxanen und Aminosäuren beruhenden Schwierigkeiten zu überwinden. Es hat daher nicht an mannigfachen Anstrengungen gefehlt, unter Nutzung verschiedenartigster chemischer Verknüpfungskonzepte Zugänge in diese interessanten Substanzklassen zu erschließen.
So beschreibt beispielsweise EP 1149855 eine mögliche Methode zur Herstellung von mit Arginin funktionalisierten Siloxanen unter Verwendung von Anhydrid funktionalisierten Siloxanen. Die Anhydridfunktionalität wird hierbei z.B. durch Hydrosilylierung von Allylsuccinanhydrid, was teuer und toxikologisch bedenklich ist, eingeführt. Anschließend wird das Siloxan mit einem Überschuss an ungeschütztem Arginin in Ethanol umgesetzt.
JP 2004-182680 beschreibt ein Kosmetikprodukt, welches ein Silicon Polymer enthält, das durch ein Aminosäurederivat modifiziert ist. Dabei bedient man sich einer aufwendigen 4-stufigen Synthese mit teils giftigen Zwischenprodukten, in der im letzten Schritt ein isocyanathaltiges Siloxan mit einer modifizierten Aminosäure gekoppelt wird. Die Endprodukte enthalten jedoch keine freien Aminogruppen.
Die US 5516869 offenbart spezielle $\alpha,\omega$-aminosäuremodifizierte Siloxane, die durch die hydrosilylierende Verknüpfung von Alkenylpyrrolidonen mit $\alpha,\omega$-SiH substituierten Siloxanen synthetisiert werden.
Einen weiteren Zugang stellt die in der US 5412074 beschriebene Reaktion von $\alpha,\omega$-epoxidmodifizierten Siloxanen mit Proteinen dar, die Produkte ergibt, deren Siloxaneinheiten über Polypeptidbrücken undefinierter Länge miteinander verbunden sind.
Einen ähnlichen Zugang beschreibt die Schrift EP 1477512 A1, in der Monoepoxysiloxane mit $\varepsilon$-Polylysin umgesetzt werden. Diese Strukturen weisen antimikrobielle Eigenschaften auf.
WO 2009084711 A1 beschreibt die Herstellung von aminosäuremodifizierten Siloxanemulsionen. Die gegensätzlichen Löslichkeitseigenschaften der epoxyhaltigen Siloxane und der ungeschützten Aminosäuren wird hier umgangen, indem die Umsetzung in Gegenwart von etwa 60 % Wasser und 5 bis 10 % Emulgatoren durchgeführt wird. Die Emulsionen wurden in kosmetischen Anwendungen getestet.
Die im Stand der Technik verwendeten Methoden zur Kupplung von Aminosäuren oder Peptiden an Siloxane bestehen unter anderem in Epoxidringöffnungsreaktionen, Ver- und Umesterungen, Amidierungen und Substitutionsreaktionen. Die Nachteile der im Stand der Technik beschriebenen teils mehrstufigen Verfahren ist der Einsatz von teilweise toxischen und schwierig zu handhabenden Rohstoffen, der Einsatz von teuren Aminosäurederivaten, die Erfordernis von hohen Temperaturen bei teils langen Reaktionszeiten, zur Verfärbung und Quervernetzung führende Nebenreaktionen und niedrige Ausbeuten wie z.B. in Reaktionen in Emulsionen.
Aufgabe der vorliegenden Erfindung ist es, ein Verfahren zur Herstellung von aminosäuremodifizierten Siloxane bereitzustellen, welches unter milden Bedingungen durchgeführt werden kann.
Eine weitere Aufgabe der vorliegenden Erfindung ist es, neue organomodifizierte Silikone für Pflegeformulierungen für Haut, Haar und Textilien bereitzustellen, die toxikologisch unbedenklich sind.

Beschreibung der Erfindung

Gegenstand der vorliegenden Erfindung ist ein Verfahren zur Herstellung von Aminosäuregruppen enthaltenden Siloxanen durch Reaktion eines organischen Aminosäuresalzes mit epoxy- und/oder carbonatgruppenhaltigen Siloxanen.
Vorteil des vorliegenden Verfahrens ist die Vermeidung teurer Aminosäure-Derivate. Ein weiterer Vorteil des erfindungsgemäßen Verfahrens ist die Vermeidung toxischer Lösungsmittel.
Ein weiterer Vorteil des erfindungsgemäßen Verfahrens ist in der kurzen Reaktionszeit der Verfahrensschritte begründet. Ferner stellt das erfindungsgemäße Verfahren eine starke Vereinfachung gegenüber den Verfahren im Stand der Technik, die teils bei hohen Temperaturen durchgeführt werden müssen, und wodurch stark gefärbte Produkte erhalten werden.

Das erfindungsgemäße Verfahren zur Herstellung von Aminosäuregruppen enthaltenden Siloxanen zeichnet sich dadurch aus, dass zunächst eine Aminosäure effizient in ein Aminosäuresalz überführt wird, und dieses anschließend mit einem epoxy- und/oder carbonatgruppenhaltigen Siloxan in Gegenwart eines geeigneten Lösungsmittels umgesetzt wird.

Gegenstand der vorliegenden Erfindung ist ein Verfahren zur Herstellung von aminosäuremodifizierten Siloxanen umfassend die Verfahrensschritte

C) Umsetzen eines Salzes der allgemeinen Formel $K^+A^-$ mit mindestens einem mindestens eine Epoxy- und/oder Carbonatgruppe aufweisenden Siloxan, wobei $K^+$ ein organisches Kation und
$A^-$ das Carboxylat einer $\alpha$-Amino-Carbonsäure, gegebenenfalls
D) Umsetzen mit einer weiteren epoxygruppenhaltigen, organischen Komponente und gegebenenfalls
E) Aufreinigen des aminosäuremodifizierten Siloxans.

Unter dem Begriff "aminosäuremodifiziertes Siloxan" ist im Zusammenhang mit der vorliegenden Erfindung ein Siloxan zu verstehen, das mindestens eine Gruppe enthält, die durch das Ausbilden einer kovalenten Bindung zu einer Aminosäure entstanden ist. Unter dem Begriff "organisches Kation" sind im Zusammenhang mit der vorliegenden Erfindung in ihrer Gesamtnettoladung positiv geladene organische Verbindungen zu verstehen.
Unter dem Begriff "Carbonatgruppe" ist im Zusammenhang mit der vorliegenden Erfindung eine Gruppe der Struktur

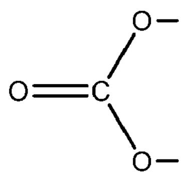

zu verstehen.

Es ist erfindungsgemäß bevorzugt, dass es sich bei dem organischen Kation $K^+$ um Verbindungen mit einer Ammonium-Gruppe (Ammonium-Kationen) oder einer Phosphonium-Gruppe (Phosphonium-Kationen) handelt.

Unter dem Begriff "Ammonium-Kationen" sind im Zusammenhang mit der vorliegenden Erfindung nicht-aromatische Verbindungen mit lokalisierter positiver Ladung am Stickstoffatom, z.B. Verbindungen mit vierbindigem Stickstoff (quaternäre Ammoniumverbindungen) oder Verbindungen mit dreibindigem Stickstoff, wobei eine Bindung eine Doppelbindung ist, oder aromatische Verbindungen mit delokalisierter positiver Ladung und mindestens einem, vorzugsweise ein bis drei Stickstoffatomen im aromatischen Ringsystem, zu verstehen.
Unter dem Begriff "Phosphonium-Kationen" sind im Zusammenhang mit der vorliegenden Erfindung nicht-aromatische Verbindungen mit lokalisierter positiver Ladung am Phophoratom, z.B. Verbindungen mit vierbindigem Phosphor (quaternäre Phosphoniumverbindungen) oder Verbindungen mit dreibindigem Phosphor, wobei eine Bindung eine Doppelbindung ist, zu verstehen.

Geeignete organische Kationen $K^+$ sind beispielsweise solche der allgemeinen Formel I:

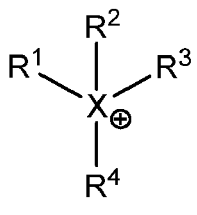

allgemeine Formel I

in denen $R^1$, $R^2$, $R^3$, $R^4$
gleich oder unterschiedlich sind und
einen linearen oder verzweigten gegebenenfalls Doppelbindungen enthaltenden aliphatischen Kohlenwasserstoffrest mit 1 bis 30 Kohlenstoffatomen,
einen gegebenenfalls Doppelbindungen enthaltenden cycloaliphatischen Kohlenwasserstoffrest mit 5 bis 40 Kohlenstoffatomen,

einen aromatischen Kohlenwasserstoffrest mit 6 bis 40 Kohlenstoffatomen, einen Alkylarylrest mit 7 bis 40 Kohlenstoffatomen,
einen durch ein oder mehrere Heteroatome (Sauerstoff, NH, NR' mit R' gleich einem gegebenenfalls Doppelbindungen enthaltenden C1 bis C30-Alkylrest, insbesondere -$CH_3$) unterbrochenen linearen oder verzweigten gegebenenfalls Doppelbindungen enthaltenden aliphatischen Kohlenwasserstoffrest mit 2 bis 30 Kohlenstoffatomen,
einen durch eine oder mehrere Funktionalitäten, ausgewählt aus der Gruppe -O-C(O)-, -(O)C-O-, -NH-C(O)-, -(O)C-NH, - ($CH_3$)N-C (O)-, -(O)C-N($CH_3$)-, -S($O_2$)-O-, -O-S($O_2$)-, -S($O_2$)-NH-, -NH-S($O_2$)-, -S ($O_2$)-N ($CH_3$)-, -N($CH_3$)-S($O_2$)-, unterbrochenen linearen oder verzweigten gegebenenfalls Doppelbindungen enthaltenden aliphatischen Kohlenwasserstoffrest mit 2 bis 30 Kohlenstoffatomen,
einen endständig OH, OR', $NH_2$, N(H)R', $N(R')_2$ (mit R' gleich einem gegebenenfalls Doppelbindungen enthaltenden C1- bis C30-Alkylrest) funktionalisierten linearen oder verzweigten gegebenenfalls Doppelbindungen enthaltenden aliphatischen oder cycloaliphatischen Kohlenwasserstoffrest mit 1 bis 30 Kohlenstoffatomen oder
einen blockweise oder statistisch aufgebauten Polyether gemäß -($R^5$-O)$_n$-$R^6$, wobei $R^5$ ein 2 bis 4 Kohlenstoffatome enthaltender linearer oder verzweigter Kohlenwasserstoffrest, n 1 bis 100, vorzugsweise 2 bis 60, ist und $R^6$ Wasserstoff, einen linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einen aromatischen Kohlenwasserstoffrest mit 6 bis 40 Kohlenstoffatomen, einen Alkylarylrest mit 7 bis 40 Kohlenstoffatomen, oder ein Rest -C(O)-$R^7$ mit $R^7$ gleich einem linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einem aromatischen Kohlenwasserstoffrest mit 6 bis 40 Kohlenstoffatomen, einem Alkylarylrest mit 7 bis 40 Kohlenstoffatomen,
bedeuten, und
X Stickstoff oder Phosphor, bevorzugt Stickstoff, ist.

Es ist insbesondere bevorzugt, dass $R^1$, $R^2$, $R^3$, $R^4$ gleich oder unterschiedlich sind und einen linearen oder verzweigten aliphatischen Kohlenwasserstoffrest mit 1 bis 30 Kohlenstoffatomen bedeuten, mit der Maßgabe, dass mindestens einer der Reste $R^1$, $R^2$, $R^3$, $R^4$ 4 bis 30 Kohlenstoffatome, bevorzugt 8 bis 26 Kohlenstoffatome, besonders bevorzugt 10 bis 22 Kohlenstoffatome, aufweist.

Weitere geeignete organische Kationen $K^+$ leiten sich von gesättigten oder ungesättigten cyclischen Verbindungen sowie von aromatischen Verbindungen mit jeweils mindestens einem 3-bindigen Stickstoffatom in einem 4- bis 10-, vorzugsweise 5-bis 6-gliedrigen heterocyclischen Ring ab, der gegebenenfalls substituiert sein kann. Solche organischen Kationen $K^+$ lassen sich vereinfacht (d. h. ohne Angabe von genauer Lage und Zahl der Doppelbindungen im Molekül) durch die nachstehende allgemeine Formel II beschrieben, wobei die heterocyclischen Ringe gegebenenfalls auch mehrere Hetereoatome enthalten können.

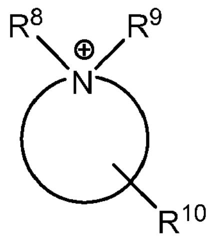

allgemeine Formel II,

mit

$R^8$ und $R^9$ gleich oder unterschiedlich wie oben genannte $R^1$, $R^2$, $R^3$, $R^4$, und
$R^{10}$ Wasserstoff, linearer oder verzweigter gegebenenfalls Doppelbindungen enthaltender aliphatischer Kohlenwasserstoffrest mit 1 bis 30 Kohlenstoffatomen,
ein cycloaliphatischer gegebenenfalls Doppelbindungen enthaltenderKohlenwasserstoffrest mit 5 bis 40 Kohlenstoffatomen,
ein aromatischer Kohlenwasserstoffrest mit 6 bis 40 Kohlenstoffatomen oder
ein Alkylarylrest mit 7 bis 40 Kohlenstoffatomen.

Beispiele für cyclische Stickstoffverbindungen der vorgenannten Art sind Pyrrolidin, Dihydropyrrol, Pyrrol, Imidazolin,

Oxazolin, Oxazol, Thiazolin, Thiazol, Isoxazol, Isothiazol, Indol, Carbazol, Piperidin, Pyridin, die isomeren Picoline und Lutidine, Chinolin und iso-Chinolin.

Die cyclischen Stickstoffverbindungen der allgemeinen Formel II können unsubstituiert ($R^{10}$ = H), einfach oder auch mehrfach durch den Rest $R^{10}$ substituiert sein, wobei bei einer Mehrfachsubstitution durch $R^{10}$ die einzelnen Reste $R^{10}$ unterschiedlich sein können.

Als weitere geeignete organische Kationen $K^+$ kommen weiterhin Ionen in Betracht, die sich von gesättigten acyclischen, gesättigten oder ungesättigten cyclischen Verbindungen sowie von aromatischen Verbindungen mit jeweils mehr als einem dreibindigen Stickstoffatom in einem 4-bis 10-, vorzugsweise 5- bis 6-gliedrigen heterocyclischen Ring ableiten. Diese Verbindungen können sowohl an den Kohlenstoffatomen als auch an den Stickstoffatomen substituiert sein. Sie können ferner durch, gegebenenfalls substituierte, Benzolringe und/oder Cyclohexanringe unter Ausbildung mehrkerniger Strukturen anelliert sein. Beispiele für solche Verbindungen sind Pyrazol, 3,5-Dimethylpyrazol, Imidazol, Benzimidazol, N-Methylimidazol, Dihydropyrazol, Pyrazolidin, Pyridazin, Pyrimidin, Pyrazin, Pyridazin, Pyrimidin, 2,3-, 2,5- und 2,6-Dimethylpyrazin, Cinnolin, Phthalazin, Chinazolin, Phenazin und Piperazin. Insbesondere vom Imidazol und seinen Alkyl- und Phenylderivaten abgeleitete organische Kationen der allgemeinen Formel III haben sich als Bestandteil organischer Salze bewährt.

Als organische Kationen $K^+$ kommen ferner Ionen in Betracht, welche zwei Stickstoffatome enthalten und durch die allgemeine Formel III wiedergegeben sind

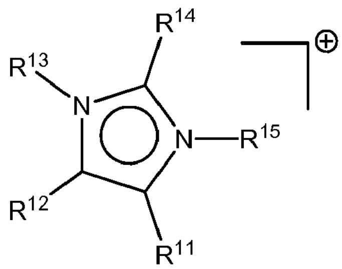

allgemeine Formel III

in denen $R^{11}$, $R^{12}$, $R^{13}$, $R^{14}$ $R^{15}$ gleich oder unterschiedlich sind und

Wasserstoff,

einen linearen oder verzweigten gegebenenfalls Doppelbindungen enthaltenden aliphatischen Kohlenwasserstoffrest mit 1 bis 30 Kohlenstoffatomen,

einen cycloaliphatischen gegebenenfalls Doppelbindungen enthaltenden Kohlenwasserstoffrest mit 5 bis 40 Kohlenstoffatomen,

einen aromatischen Kohlenwasserstoffrest mit 6 bis 40 Kohlenstoffatomen, einen Alkylarylrest mit 7 bis 40 Kohlenstoffatomen,

einen durch ein oder mehrere Heteroatome (Sauerstoff, NH, NR' mit R' gleich einem gegebenenfalls Doppelbindungen enthaltenden $C_1$ bis $C_{30}$-Alkylrest), unterbrochenen linearen oder verzweigten gegebenenfalls Doppelbindungen enthaltenden aliphatischen Kohlenwasserstoffrest mit 1 bis 30 Kohlenstoffatomen,

einen durch ein oder mehrere Funktionalitäten, ausgewählt aus der Gruppe -O-C(O)-, - (O)C-O-, -NH-C(O)-, -(O)C-NH, $-(CH_3)N-C(O)-$, $-(O)C-N(CH_3)-$, $-S(O_2)-O-$, $-O-S(O_2)-$, -S $(O_2)$-NH-, -NH-S $(O_2)$-, -S $(o_2)-N(CH_3)-$, $-N(CH_3)-S(O_2)-$, unterbrochenen linearen oder verzweigten gegebenenfalls Doppelbindungen enthaltenden aliphatischen Kohlenwasserstoffrest mit 1 bis 30 Kohlenstoffatomen,

einen endständig OH, OR', $NH_2$, N(H)R', $N(R')_2$ mit R' gleich einem gegebenenfalls Doppelbindungen enthaltenden $C_1$- bis $C_{30}$-Alkylrest, funktionalisierten linearen oder verzweigten gegebenenfalls Doppelbindungen enthaltenden aliphatischen oder cycloaliphatischen Kohlenwasserstoffrest mit 1 bis 30 Kohlenstoffatomen oder

einen blockweise oder statistisch aufgebauten Polyether aufgebaut aus $-(R_5-O)_n-R_6$ bedeuten, wobei $R^5$ ein 2 bis 4 Kohlenstoffatome enthaltender Kohlenwasserstoffrest, n 1 bis 100 ist und $R^6$ Wasserstoff, einen linearen oder verzweigten gegebenenfalls Doppelbindungen enthaltenden aliphatischen Kohlenwasserstoffrest mit 1 bis 30 Kohlenstoffatomen, einen cycloaliphatischen gegebenenfalls Doppelbindungen enthaltenden Kohlenwasserstoffrest mit 5 bis 40 Kohlenstoffatomen, einen aromatischen Kohlenwasserstoffrest mit 6 bis 40 Kohlenstoffatomen, einen Alkylarylrest mit 7 bis 40 Kohlenstoffatomen bedeutet oder einen Rest $-C(O)-R^7$ mit $R^7$ gleich einem linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einem aromatischen Kohlenwasserstoffrest mit 6 bis 40 Kohlenstoffatomen, einem Alkylarylrest mit 7 bis 40 Kohlenstoffatomen, darstellen.

Als das Carboxylat einer α-Amino-Carbonsäure $A^-$ können alle Carboxylate eingesetzt werden, bei denen sich in alpha-Stellung zu der Carboxy-Gruppe eine Aminogruppe befindet. Solche Verbindungen können beispielsweise Aminosäuren, Proteine, Peptide oder Oligo-Peptide umfassen.
Als das Carboxylat einer α-Amino-Carbonsäure $A^-$ werden insbesondere Carboxylate der α-Amino-Carbonsäuren ausgewählt aus L-α-Amino-Carbonsäuren, bevorzugt ausgewählt aus den 22 proteinogenen Aminosäuren, welche gegebenenfalls glykosyliert sein können, ausgewählt aus Glycin, Alanin, Valin, Leucin, Isoleucin, Prolin, Methionin, Phenylalanin, Tyrosin, Tryptophan, Serin, Threonin, Asparagin, Glutamin, Cystein, Lysin, Arginin, Histidin, Aspartat, Selenocystein, Pyrrolysin und Glutamat, insbesondere bevorzugt Lysin, Arginin und Histidin, eingesetzt.
Als das Carboxylat einer a-Amino-Carbonsäure $A^-$ können auch Carboxylate von Sarcosin, γ-Aminobuttersäure, Ornithine, Creatin, Opin, Cystine, Hydroxyprolin, Hydroxylysine, Thyroxin und O-Phophoserin eingesetzt werden.
In dem erfindungsgemäßen Verfahren können mindestens eine Epoxy- und/oder Carbonatgruppe aufweisende Siloxane eingesetzt werden, bei denen die eine Epoxy- und/oder Carbonatgruppe rein endständig, rein seitenständig oder gemischt end- und seitenständig im Siloxan angeordnet sind. Es können auch mindestens eine Epoxy- und/oder Carbonatgruppe aufweisende cyclische Siloxane eingesetzt werden.
In dem erfindungsgemäßen Verfahren werden bevorzugt mindestens eine Epoxy- und/oder Carbonatgruppe aufweisende Siloxane der allgemeinen Formel IV

$$M_{a1}M^{A}_{a2}M^{B}_{a3}D_{b1}D^{A}_{b2}D^{B}_{b3}T_{c1}T^{A}_{c2}T^{B}_{c3}Q_{d1}$$ allgemeine Formel IV

mit

$M = [R^{16}{}_3SiO_{1/2}]$
$M^A = [R^{17}R^{16}{}_2SiO_{1/2}]$
$M^B = [R^{18}R^{16}{}_2SiO_{1/2}]$
$D = [R^{16}{}_2SiO_{2/2}]$
$D^A = [R^{17}{}_1R^{16}{}_1SiO_{2/2}]$
$D^B = [R^{1B}{}_1R^{16}{}_1SiO_{2/2}]$
$T = [R^{16}SiO_{3/2}]$
$T^A = [R^{17}SiO_{3/2}]$
$T^B = [R^{1B}SiO_{3/2}]$
$Q = [SiO_{4/2}]$,

wobei gilt

$R^{16}$ unabhängig voneinander gleiche oder verschiedene lineare oder verzweigte, gesättigte oder ungesättigte Kohlenwasserstoffreste mit 1 bis 30 Kohlenstoffatomen oder auch aromatische Kohlenwasserstoffreste mit 6 bis 30 Kohlenstoffatomen bevorzugt Methyl oder Phenyl, insbesondere Methyl,
$R^{17}$ unabhängig voneinander gleiche oder verschiedene epoxy- und/oder carbonatgruppenhaltige Reste,
$R^{18}$ unabhängig voneinander gleiche oder verschiedene lineare oder verzweigte, gesättigte oder olefinisch ungesättigte Kohlenwasserstoffreste mit 8 bis 30 Kohlenstoffatomen, beispielsweise Decyl-, Dodecyl, Tetradecyl-, Hexadecyl-, Octadecyl-, insbesondere Hexadecyl- und Octadecyl-,
einen aromatischen Kohlenwasserstoffrest mit 6 bis 40 Kohlenstoffatomen, einen Alkylarylrest mit 7 bis 40 Kohlenstoffatomen,
einen durch ein oder mehrere Heteroatome (Sauerstoff, NH, NR' mit R' gleich einem gegebenenfalls Doppelbindungen enthaltenden C1 bis C30-Alkylrest, insbesondere $-CH_3$) unterbrochenen linearen oder verzweigten gegebenenfalls Doppelbindungen enthaltenden aliphatischen Kohlenwasserstoffrest mit 2 bis 30 Kohlenstoffatomen,
einen durch eine oder mehrere Funktionalitäten, ausgewählt aus der Gruppe -O-C(O)-, -(O)C-O-, -NH-C(O)-, -(O)C-NH, - $(CH_3)$N-C (O)-, -(O)C-N$(CH_3)$-, -S$(O_2)$-O-, -O-S$(O_2)$-, -S$(O_2)$-NH-, -NH-S$(O_2)$-, -S $(O_2)$-N $(CH_3)$-, -N$(CH_3)$-S$(O_2)$-, unterbrochenen linearen oder verzweigten gegebenenfalls Doppelbindungen enthaltenden aliphatischen Kohlenwasserstoffrest mit 2 bis 30 Kohlenstoffatomen,
einen endständig OH, OR', $NH_2$, N(H)R', $N(R')_2$ (mit R' gleich einem gegebenenfalls Doppelbindungen enthaltenden C1- bis C30-Alkylrest) funktionalisierten linearen oder verzweigten gegebenenfalls Doppelbindungen enthaltenden aliphatischen oder cycloaliphatischen Kohlenwasserstoffrest mit 1 bis 30 Kohlenstoffatomen oder
einen blockweise oder statistisch aufgebauten Polyether gemäß $-(R^5\text{-}O)_n\text{-}R^6$, wobei $R^5$ ein 2 bis 4 Kohlenstoffatome enthaltender linearer oder verzweigter Kohlenwasserstoffrest, n 1 bis 100, vorzugsweise 2 bis 60, ist und $R^6$ Wasserstoff, einen linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einen aromatischen Kohlenwasserstoffrest mit 6 bis

40 Kohlenstoffatomen, einen Alkylarylrest mit 7 bis 40 Kohlenstoffatomen, oder ein Rest -C(O)-$R^7$ mit $R^7$ gleich einem linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einem aromatischen Kohlenwasserstoffrest mit 6 bis 40 Kohlenstoffatomen, einem Alkylarylrest mit 7 bis 40 Kohlenstoffatomen,

a1 = 0 bis 200, bevorzugt 1 bis 60, insbesondere 0,
a2 = 0 bis 30, bevorzugt 1 bis 20, insbesondere 2 bis 10,
a3 = 0 bis 30, bevorzugt 1 bis 20, insbesondere 0,
b1 = 3 bis 5000, bevorzugt 3 bis 1000, insbesondere 10 bis 500,
b2 = 0 bis 100, bevorzugt 1 bis 30, insbesondere 1 bis 10,
b3 = 0 bis 100, bevorzugt 0 bis 30, insbesondere 0,
c1 = 0 bis 30, bevorzugt 1 bis 30, alternativ bevorzugt 0,
c2 = 0 bis 30, bevorzugt 0 bis 10, insbesondere 0 bis 5,
c3 = 0 bis 30, bevorzugt 0 bis 5, insbesondere 0,
d1 = 0 bis 30, bevorzugt 1 bis 5, alternativ bevorzugt 0,

mit der Maßgabe, dass mindestens einer der Indices a2, b2 oder c2 # 0 eingesetzt.

Erfindungsgemäß bevorzugte im Verfahren eingesetzte mindestens eine Epoxygruppe aufweisende Siloxane sind gekennzeichnet durch die Parameterkennzeichnung ausgewählt aus der Gruppe:

a1 = 0, a2= 2, a3 = 0, b1 = 5-350, b2 = 0, b3 = 0, c1= 0, c2 = 0, c3 = 0 und d1 = 0,
a1 = 3-12, a2= 0, a3 = 0, b1 = 15-350, b2 = 0, b3 = 0, c1= 0, c2 = 1-10, c3 = 0 und d1 = 0,
a1 = 2, a2= 0, a3 = 0, b1 = 10-350, b2 = 1-30, b3 = 0, c1= 0, c2 = 0, c3 = 0 und d1 = 0,
a1 = 0, a2= 2, a3 = 0, b1 = 10-350, b2 = 1-30, b3 = 0, c1= 0, c2 = 0, c3 = 0 und d1 = 0,
a1 = 0, a2= 3-12, a3 = 0, b1 = 15-350, b2 = 0, b3 = 0, c1= 1-10, c2 = 0, c3 = 0 und d1 = 0,
a1 = 0, a2= 4-22, a3 = 0, b1 = 20-350, b2 = 0, b3 = 0, c1= 0, c2 = 0, c3 = 0 und d1 = 1-10,
a1 = 2-11, a2= 2-11, a3 = 0, b1 = 20-350, b2 = 0, b3 = 0, c1= 0, c2 = 0, c3 = 0 und d1 = 1-10,
a1 = 2-11, a2= 2-11, a3 = 0, b1 = 20-350, b2 = 1-10, b3 = 0, c1= 0, c2 = 0, c3 = 0 und d1 = 1-10,
a1 = 0, a2= 3-12, a3 = 0, b1 = 15-350, b2 = 1-10, b3 = 0, c1= 1-10, c2 = 0, c3 = 0 und d1 = 0,
a1 = 3-12, a2= 0, a3 = 0, b1 = 15-350, b2 = 1-10, b3 = 0, c1= 0, c2 = 1-10, c3 = 0 und d1 = 0,
a1 = 0, a2= 5-17, a3 = 0, b1 = 30-350, b2 = 0, b3 = 0, c1= 1-5, c2 = 0, c3 = 0 und d1 = 1-5 und
a1 = 0, a2= 0, a3 = 0, b1 = 0-10, b2 = 1-10, b3 = 0, c1= 0, c2 = 0, c3 = 0 und d1 = 0.

Es ist erfindungsgemäß bevorzugt, dass in dem Verfahren mindestens eine Epoxy- und/oder Carbonatgruppe aufweisende Siloxane eingesetzt werden, bei denen die epoxygruppenhaltigen Reste $R^{17}$ der allgemeinen Formel Va und die carbonatgruppenhaltigen Reste $R^{17}$ der allgemeinen Formel Vb entsprechen

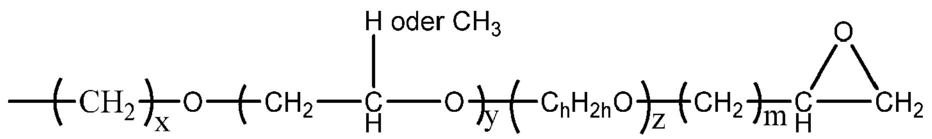

allgemeine Formel Va

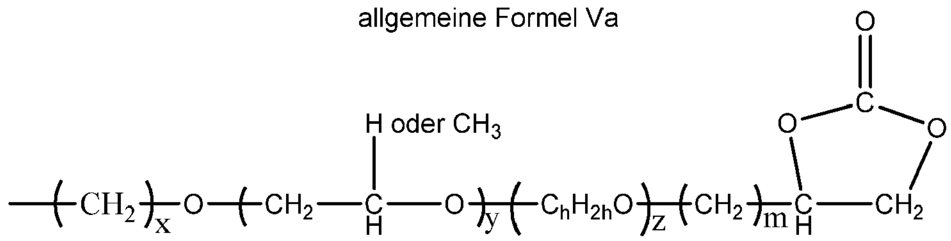

allgemeine Formel Vb

wobei m = 1-20, x = 1-20, y = 0-10, z = 0-50 und h = 1-10.

In Verfahrensschritt C) kann erfindungsgemäß ein Lösungsmittel eingesetzt werden, wie beispielsweise Wasser, Aceton, Acetonitril, tert.-Butanol, Chloroform, Dichlormethan, Essigsäure, Bis(2-methoxyethyl)ether, Dimethylacetamide, Ethanol, Ethylenglycol, Dipropylenglycol, Methanol, Isopropanol, Diethylether, Pyridin, Dimethylsulfoxid, Dimethylformamid, Polyether und deren Mischungen. Insbesondere bevorzugt werden in Verfahrensschritt C) protische Lösungsmittel eingesetzt, die mindestens teilweise Wasser enthalten können.

Bevorzugt liegt der pH-Wert des Lösungsmittels in Verfahrensschritt C) bei 25 °C in einem Bereich von 1 bis 14, bevorzugt von 3 bis 9, insbesondere von 5 bis 7.

Es kann in einer alternativen aber nicht weniger bevorzugten Ausführungsform in Verfahrensschritt C) mit mindestens zwei Lösungsmitteln gearbeitet werden, die ein mehrphasiges System bilden.

Solche, mindestens zwei, ein mehrphasiges System bildenden Lösungsmitteln enthaltende Systeme beinhalten beispielsweise mindestens eine Komponente ausgewählt aus Wasser, Chloroform, Diethylether, Dichlormethan, Toluol und Xylol.

Verfahrensschritt C) des erfindungsgemäßen Verfahrens kann in einem Temperaturbereich von 20 bis 200 °C durchgeführt werden, vorzugsweise von 40 bis 120 °C, insbesondere bevorzugt von 60 bis 100 °C.

Verfahrensschritt C) des erfindungsgemäßen Verfahrens kann in einem Druckbereich von 0 bis 20 bar, vorzugsweise 0 bis 2 bar, insbesondere bevorzugt bei 0,9 bis 1,1 bar durchgeführt werden.

Verfahrensschritt C) des erfindungsgemäßen Verfahrens kann sowohl unter Inertisierung mit Edelgasen wie z. B. Argon oder auch unter Stickstoff oder unter gewöhnlicher Atmosphäre durchgeführt werden. Besonders bevorzugt ist die Durchführung unter Inertgas, wobei Stickstoff besonders bevorzugt ist.

Die Reaktionsmischung kann durch beliebiges Mischen der Komponenten erhalten werden. Dabei spielt es keine Rolle, welche Komponente in welcher Reihenfolge zugegeben wird.

Verfahrensschritt C) des erfindungsgemäßen Verfahrens kann sowohl als Eintopfverfahren (Batch-Verfahren) als auch über Zudosage der Rohstoffe durchgeführt werden. Bei Letzterem legt man bevorzugt das Salz der allgemeinen Formel $K^+A^-$ vor und dosiert das mindestens eine Epoxygruppe aufweisende Siloxan gegebenenfalls in einem Lösungsmittel über einen Zeitraum von 0,5 - 2 Stunden zu. Besonders bevorzugt werden zunächst die Ausgangsstoffe und gegebenenfalls Lösungsmittel vermischt.

Erfindungsgemäß bevorzugt ist eine Dauer des Verfahrensschrittes C) von weniger als zehn Stunden.

Verfahrensschritt C) des erfindungsgemäßen Verfahrens wird bevorzugt im Batch-Verfahren durchgeführt.

Weiterhin lässt sich das entstandene aminosäurehaltige Siloxan mit einer weiteren epoxyhaltigen, organischen Komponente in Verfahrensschritt D) umsetzen.

Die organischen, epoxidischen Verbindungen können dabei monomerer oder polymerer Natur sein. Bevorzugt sind dabei polyetherhaltige und aromatische Epoxide.

Die Umsetzung in Verfahrensschritt D) gleicht in ihren Bedingungen der Umsetzung in Verfahrensschritt C).

Es ist erfindungsgemäß bevorzugt, wenn das Salz der allgemeinen Formel $K^+A^-$ für Verfahrensschritt C) des erfindungsgemäßen Verfahrens über die zusätzlichen Verfahrensschritte A) und B) wie im Folgenden beschrieben bereit gestellt wird. Somit ist ein bevorzugtes erfindungsgemäßes Verfahren dadurch gekennzeichnet, dass es die zusätzlichen Verfahrensschritte umfasst

A) Umsetzen einer $\alpha$-Amino-Carbonsäure $H^+A^-$ mit einem organischen Salz eines organischen Kations $K^+$ in Anwesenheit einer Base zu einem Salz der allgemeinen Formel $K^+A^-$,
und gegebenenfalls
B) Aufreinigen des Salzes der allgemeinen Formel $K^+A^-$.

Es ist offenbar, dass als in Verfahrensschritt A) einzusetzende "$\alpha$-Amino-Carbonsäure $H^+A^-$" die korrespondierende Säure des "Carboxylats einer $\alpha$-Amino-Carbonsäure $A^-$" des Verfahrensschrittes C) eingesetzt werden muss. Die $\alpha$-Amino-Carbonsäure $H^+A^-$ kann in Verfahrensschritt A) in Form ihrer Addukte und/oder Salze, wie beispielsweise Hydrate, Hydrochloride und Acetate, eingesetzt werden. Diese können auch in Form einer wässrigen Lösung eingesetzt werden.

Die als in Verfahrensschritt A) einzusetzende "$\alpha$-Amino-Carbonsäure $H^+A^-$" ergibt sich somit aus der Kombination des in Verfahrensschritt C) genannten "Carboxylats einer $\alpha$-Amino-Carbonsäure $A^-$" mit einem Proton, wobei oben als "bevorzugt genannte "$\alpha$-Amino-Carbonsäuren $H^+A^-$" des Verfahrensschrittes C)" bevorzugt mit einem Proton kombiniert in Verfahrensschritt A) als "$\alpha$-Amino-Carbonsäure $H^+A^-$" eingesetzt werden. Es ist offenbar, dass als in Verfahrensschritt A) einzusetzendes "organisches Salz eines organischen Kations $K^+$" das korrespondierende Salz des in Verfahrensschritt C) genannten "organischen Kations $K^+$" eingesetzt werden muss.

Das als in Verfahrensschritt A) einzusetzende "organische Salz eines organischen Kations $K^+$" ergibt sich somit aus der Kombination des in Verfahrensschritt C) genannten "organischen Kations $K^+$" mit einem Anion, wobei oben als "bevorzugt genannte organische Kationen $K^+$ des Verfahrensschrittes C)" bevorzugt mit einem Anion kombiniert in Verfahrensschritt A) als "organisches Salz eines organischen Kations $K^+$" eingesetzt werden.

Bevorzugte Gegenanionen des organischen Salzes des organischen Kations $K^+$ in Verfahrensschritt A) in diesem Zusammenhang sind ausgewählt aus der Gruppe Halogenide, Hydroxide, Bis(perfluoralkylsulfonyl)amide, Alkyl- und Aryltosylate, Perfluoralkyltosylate, Nitrate, Sulfate, Hydrogensulfate, Alkyl- und Arylsulfate, Polyethersulfate und -sulfonate, Perfluoralkylsulfate, Sulfonate, Alkyl- und Arylsulfonate, perfluorierte Alkyl- und Arylsulfonate, Alkyl- und Arylcarboxylate, Perfluoralkylcarboxylate, Perchlorate, Tetrarchloroaluminate, Saccharinate, Dicyanamid, Tetrafluoroborat, Hexafluorophosphat, Polyether-Phosphate und Phosphat, wobei Hydroxide erfindungsgemäß bevorzugt sind.

Als Base können in Verfahrensschritt A) organische und anorganische Basen eingesetzt werden. Beispiele für anorganische Basen sind Alkali- und Erdalkalimetallhydroxide wie Natriumhydroxid, Kaliumhydroxid, Calciumhydroxid, Bariumhydroxid usw.. Beispiele für organische Basen sind Alkoholate der Alkali- oder Erdalkalimetalle, Ammonium- und Phosphoniumhydroxide. Bevorzugt werden Alkali- und Erdalkalihydroxide eingesetzt.

Verfahrensschritt A) des erfindungsgemäßen Verfahrens kann erfindungsgemäß in Anwesenheit eines Lösungsmittels durchgeführt werden. Solche sind beispielsweise Wasser, Aceton, Acetonitril, tert.-Butanol, Chloroform, Dichlormethan, Essigsäure, Bis(2-methoxyethyl)ether, Dimethylacetamide, Ethanol, Ethylenglycol, Methanol, Isopropanol, Diethylether, Pyridin, Dimethylsulfoxid, Dimethylformamid, Polyether und deren Mischungen.

In Verfahrensschritt A) des erfindungsgemäßen Verfahrens werden bevorzugt protische Lösungsmittel eingesetzt, die mindestens teilweise Wasser enthalten können. Insbesondere kann das Lösungsmittel Wasser sein.

Bevorzugt liegt der pH-Wert des Lösungsmittels in Verfahrensschritt A) bei 25 °C in einem Bereich von 1 bis 14, bevorzugt 3 bis 14, insbesondere 5 bis 12.

Verfahrensschritt A) des erfindungsgemäßen Verfahrens kann in einem Temperaturbereich von 20 bis 200 °C durchgeführt werden, vorzugsweise von 20 bis 120 °C, insbesondere bevorzugt von 40 bis 100 °C.

Verfahrensschritt A) des erfindungsgemäßen Verfahrens kann in einem Druckbereich von 0 bis 20 bar, vorzugsweise 0 bis 2 bar, insbesondere bevorzugt bei 0,9 bis 1,1 bar durchgeführt werden.

Verfahrensschritt A) des erfindungsgemäßen Verfahrens kann sowohl unter Inertisierung mit Edelgasen wie z. B. Argon oder auch unter Stickstoff oder unter gewöhnlicher Atmosphäre durchgeführt werden. Besonders bevorzugt ist die Durchführung unter Inertgas, wobei Stickstoff besonders bevorzugt ist.

Die Reaktionsmischung kann durch beliebiges Mischen der Komponenten erhalten werden. Dabei spielt es keine Rolle, welche Komponente in welcher Reihenfolge zugegeben wird.

Verfahrensschritt A) des erfindungsgemäßen Verfahrens kann sowohl als Eintopfverfahren (Batch-Verfahren) als auch über Zudosierung der Rohstoffe durchgeführt werden.

Erfindungsgemäß bevorzugt ist eine Dauer des Verfahrensschritt B) des erfindungsgemäßen Verfahrens von 2 bis 24 Stunden.

Es kann vorteilhaft sein, wenn in Verfahrensschritt B) des erfindungsgemäßen Verfahrens Nebenprodukte aus dem Reaktionsgemisch abgetrennt werden. Solche Nebenprodukte können insbesondere solche sein, die durch Umsalzung erhalten werden. Bevorzugt sind solche Nebenprodukte Alkali- und Erdalkalihalogenide, was z.B. durch den Einsatz von Alkalihydroxiden als Base und quartäre Ammoniumhalogenide als Austauschsalz erreicht wird. Die Entfernung der Nebenprodukte, insbesondere der Alkali- und Erdalkalihalogenide kann z. B, durch eine einfache Filtration erfolgen. Der Feststoff kann auf bekannte Art und Weise aus dem Reaktionsgemisch entfernt werden. Vorzugsweise wird der Feststoff durch Filtration aus dem Reaktionsgemisch getrennt. Die Filtration kann sowohl mit als auch ohne Anlegen eines Vakuums ausgeführt werden. Als Filtermaterialien können z. B. cellulosehaltige Tiefenfilter eingesetzt werden. Vorzugsweise werden Plattenfilter mit Cellulose, Perlit, Kieselgur, Zeolith und Aktivkohle als Filtermaterial verwendet.

Weiterhin kann es notwendig sein, das eingesetzte Lösungsmittel in Verfahrensschritt B) des erfindungsgemäßen Verfahrens zu entfernen. Dazu bedient man sich einer einfachen Destillation des Lösungsmittels.

Im Falle von Wasser kann alternativ auch eine Gefriertrocknung in Verfahrensschritt B) des erfindungsgemäßen Verfahrens durchgeführt werden.

Die Destillation in Verfahrensschritt B) des erfindungsgemäßen Verfahrens erfolgt vorzugsweise bei einer Sumpftemperatur (Temperatur des Reaktionsgemisches) von 50 °C bis 200 °C, bevorzugt 50 °C bis 150 °C, insbesondere bevorzugt 80 °C bis 120 °C und einem Druck von 0,1 bis 1013 mbar, bevorzugt >0,1 bis 100 mbar, insbesondere >0,1 bis 20 mbar.

Ein weiterer Gegenstand der vorliegenden Erfindung ist das Zwischenprodukt des erfindungsgemäßen Verfahrens, wie im Folgenden beschrieben. Dieses ist insbesondere vorteilhaft geeignet, in Verfahrensschritt C) umgesetzt zu werden:

Dieser weitere Gegenstand der vorliegenden Erfindung ist somit

ein Salz der allgemeinen Formel $K^+A^-$ mit

$K^+$ ein organisches Kation und

$A^-$ das Carboxylat einer $\alpha$-Amino-Carbonsäure, dadurch gekennzeichnet, dass

$K^+$ eine Verbindungen enthaltend eine Ammonium-Gruppe darstellt, die mindestens einen organischen Rest mit 8 bis 30 Kohlenstoffatomen, bevorzugt 12 bis 26 Kohlenstoffatomen, besonders bevorzugt 16 bis 22 Kohlenstoffatomen, aufweist.

Bevorzugte erfindungsgemäße Salze sind solche, die als

organisches Kation $K^+$

Verbindungen der allgemeinen Formel I mit X = Stickstoff, II oder III,

wie oben im Zusammenhang mit dem erfindungsgemäßen Verfahren beschrieben, aufweisen,

Besonders bevorzugte erfindungsgemäße Salze sind dadurch gekennzeichnet, dass das organisches Kation $K^+$ eine Verbindung der allgemeinen Formel I

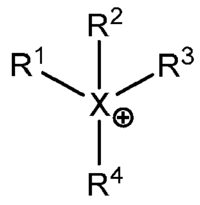

allgemeine Formel I

mit X = Stickstoff und
$R^1$, $R^2$, $R^3$, $R^4$ gleich oder unterschiedlich, linearer oder verzweigter aliphatischer Kohlenwasserstoffrest mit 1 bis 30 Kohlenstoffatomen, mit der Maßgabe, dass mindestens einer der Reste $R^1$, $R^2$, $R^3$, $R^4$ 4 bis 30 Kohlenstoffatome, bevorzugt 8 bis 26 Kohlenstoffatome, besonders bevorzugt 10 bis 22 Kohlenstoffatome, aufweist, ist.

Bevorzugte erfindungsgemäße Salze sind solche, die als
das Carboxylat einer α-Amino-Carbonsäure $A^-$
Carboxylate der Säuren ausgewählt aus L-α-Amino-Carbonsäuren, bevorzugt ausgewählt aus den 22 proteinogenen Aminosäuren, welche gegebenenfalls glykosyliert sein können, ausgewählt aus Glycin, Alanin, Valin, Leucin, Isoleucin, Prolin, Methionin, Phenylalanin, Tyrosin, Tryptophan, Serin, Threonin, Asparagin, Glutamin, Cystein, Lysin, Arginin, Histidin, Aspartat, Selenocystein, Pyrrolysin und Glutamat, insbesondere bevorzugt Lysin, Arginin und Histidin, aufweisen.
Auch Salze, die als
das Carboxylat einer α-Amino-Carbonsäure $A^-$
Carboxylate der Säuren ausgewählt aus Sarcosin, γ-Aminobuttersäure, Ornithine, Creatin, Opin, Cystine, Hydroxyprolin, Hydroxylysine, Thyroxin und O-Phophoserin aufweisen, sind alternativ bevorzugt.

Ein weiterer Gegenstand ist ein Siloxan erhältlich nach dem erfindungsgemäßen Verfahren.

Alternative erfindungsgemäße Siloxane sind insbesondere gekennzeichnet durch die allgemeine Formel VI

$$M_{a1}\,M^{C}_{a2}M^{B}_{a3}D_{b1}D^{C}_{b2}D^{B}_{b3}T_{c1}T^{C}_{c2}T^{B}_{c3}Q_{d1} \qquad \text{allgemeine Formel VI}$$

mit

$M$ = $[R^{16}_3SiO_{1/2}]$
$M^C$ = $[R^{19}R^{16}_2SiO_{1/2}]$
$M^B$ = $[R^{18}R^{16}_2SiO_{1/2}]$
$D$ = $[R^{16}_2SiO_{2/2}]$
$D^C$ = $[R^{19}_1R^{16}_1SiO_{2/2}]$
$D^B$ = $[R^{18},R^{16}_1SiO_{2/2}]$
$T$ = $[R^{16}SiO_{3/2}]$
$T^C$ = $[R^{19}SiO_{3/2}]$
$T^B$ = $[R^{18}SiO_{3/2}]$
$Q$ = $[SiO_{4/2}]$,

wobei gilt

a1, a2, a3, b1, b2, b3, c1, c2, c3, d1, $R^{16}$ und $R^{18}$ wie oben definiert,
$R^{19}$ unabhängig voneinander gleiche oder verschiedene Reste der allgemeinen Formel VIIa und/oder VIIb

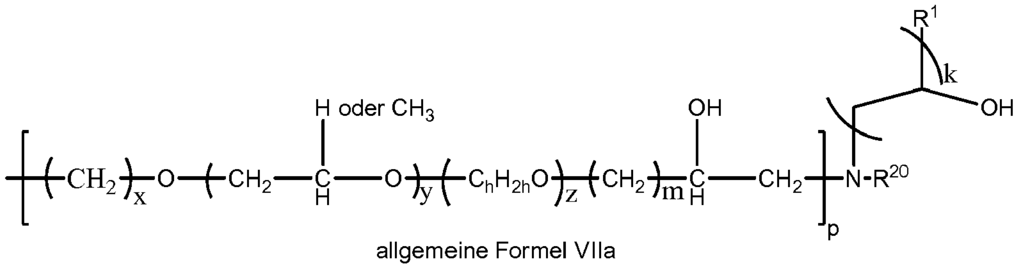

allgemeine Formel VIIa

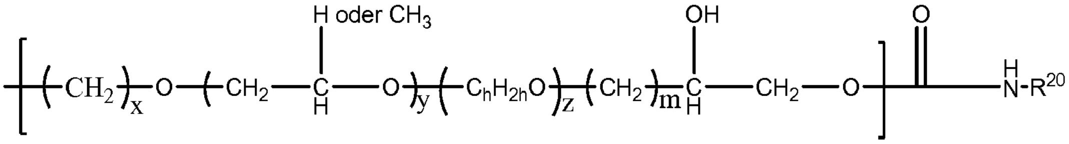

allgemeine Formel VIIb

mit p = 1-2,
k = 0-1,
x, y, z, h und m wie für die allgemeinen Formeln Va und Vb definiert,
$R^1$ wie oben definiert,

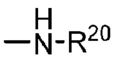 sich ableitet von oben definiertem $K^+A^-$, welches unter Verlust eines H an einer Aminogruppe mit dem Rest $R^{19}$ gebunden ist und

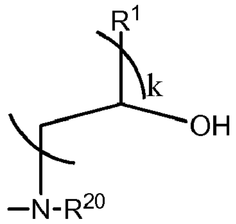

sich ableitet von

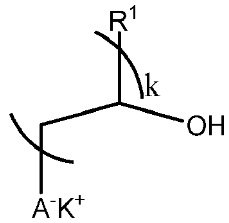

mit $K^+A^-$, wie oben definiert, welches unter Verlust eines H an einer Aminogruppe mit dem Rest $R^{19}$ gebunden ist.

Der Vollständigkeit wegen sei erwähnt, dass es sich bei dem die kovalente Bindung ausbildenden Aminogruppe nicht zwangsläufig um die alpha Aminogruppe handeln muss, so kann wie z.B. im Falle von Lysin auch die epsilon-Aminogruppe die entsprechende Bindung ausbilden. Prinzipiell kann es daher auch bei Vorliegen von zwei oder mehr Aminogruppen in $A^-$ zu Vernetzungsreaktionen kommen.
Bevorzugte Reste $R^{20}$ ergeben sich aus den oben für das erfindungsgemäße Verfahren beschriebenen bevorzugten $K^+$ und $A^-$.
Bevorzugte Parameter und Parameterkombinationen für a1, a2, a3, b1, b2, b3, c1, c2, c3, $d^1$, $R^{16}$ und $R^{18}$ sind solche wie oben für das erfindungsgemäße Verfahren beschriebene.
Ein weiterer Gegenstand der vorliegenden Erfindung ist die Verwendung der erfindungsgemäßen Siloxane und/oder der Siloxane erhältlich nach dem erfindungsgemäßen Verfahren zur Herstellung von Formulierungen, insbesondere von kosmetischen oder pharmazeutischen Formulierungen und Pflege- und Reinigungsformulierungen für die

Anwendung im häuslichen und industriellen Umfeld. In diesem Zusammenhang sind bevorzugte kosmetische oder pharmazeutische Formulierungen insbesondere Haut- und Haarbehandlungsformulierungen, insbesondere Haarkonditionierungsformulierungen. Bevorzugte Pflege- und Reinigungsformulierungen für die Anwendung im häuslichen und industriellen Umfeld sind in diesem Zusammenhang Textilpflegemittel, wie beispielsweise Weichspüler, und Pflegemittel für harte Oberflächen, insbesondere für Fahrzeuge, Wasserfahrzeuge, Flugzeuge, Fensterscheiben und -bänke, Duschabtrennungen, Fußböden wie Teppiche, Fliesen, Laminate, Parkett, Korkfußböden, Marmor-, Stein- und Feinsteinzeugböden, Haushaltskeramiken wie WCs, Waschbecken, Bidets, Duschtassen, Badewannen, Türklinken, Armaturen, Haushaltswerkzeuge wie Waschmaschinen, Trockner, Spülmaschinen, Spülen aus Keramik oder Edelstahl, Möbel wie Tische, Stühle, Regale, Ablageflächen, Fenster, Kochgeschirr, Geschirr und Besteck, Werkzeuge wie chirurgische Instrumente, Staubsauger, Maschinen, Rohrleitungen, Tanks und Geräte für Transport, Verarbeitung und Aufbewahrung in der Lebensmittelverarbeitung, wie beispielsweise *rinse aids,* Klarspüler.
Somit sind Formulierungen, insbesondere kosmetische oder pharmazeutische Formulierungen und Pflege- und Reinigungsformulierungen für die Anwendung im häuslichen und industriellen Umfeld enthaltend Siloxane erhältlich nach dem erfindungsgemäßen Verfahren, insbesondere in einer Menge von 0,1 bis 7 Gew.-%, bevorzugt 0,5 bis 4 Gew.-%, besonders bevorzugt 1 bis 3 Gew.-% bezogen auf die Gesamtformulierung, insbesondere wässrige Formulierungen, welche vorzugweise einen pH-Wert von 3,5 bis 5,5 aufweisen, ein weiterer Gegenstand der vorliegenden Erfindung.
Bevorzugte erfindungsgemäße Formulierungen enthalten keine weiteren Siloxane. Unter dem Begriff "wässrig" wird in diesem Zusammenhang ein Wassergehalt von größer als 50 Gew.-%, bevorzugt größer 75 Gew.-%, bezogen auf die Gesamtformulierung verstanden.

Ein weiterer Gegenstand der vorliegenden Erfindung ist die Verwendung der erfindungsgemäßen Siloxane und/oder der Siloxane erhältlich nach dem erfindungsgemäßen Verfahren oder der erfindungsgemäßen Formulierungen zur Konditionierung einer Oberfläche, bevorzugt von Fasern oder Geweben, insbesondere von Haut, Haaren oder Textilien.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Beispiele:

*Herstellung der erfindungsgemäßen aminosäure- und peptidmodifizierten Siloxane:*

Die Aufnahme und Interpretation der NMR-Spektren ist dem Fachmann bekannt. Als Referenz sei hiermit eingeführt das Buch "NMR Spectra of Polymers and Polymer Additives" von A. Brandolini und D. Hills, erschienen im Jahr 2000 beim Verlag Marcel Dekker Inc..

GPC-Messungen zur Bestimmung der Polydispersität und mittleren Molmassen Mw wurden unter den folgenden Messbedingungen durchgeführt: Säulenkombination SDV 1000/10000 Å (Länge 65 cm), Temperatur 30 °C, THF als mobile Phase, Fließrate 1 ml/min, Probenkonzentration 10 g/l, RI-Detektor, die Auswertung der Polymere erfolgte gegen Polystyrol-Standard (162-2570000 g/mol).

*Synthesebeispiel S1: Herstellung von Cetyltrimethylammoniumarginat*

74,3 g (0,42 mol) Arginin (Firma SAFC, 98,5%ig) wurden im 1 L-Vierhalskolben mit Innenthermometer, KPG-Rührer und Kugelkühler in Wasser suspendiert. Anschließend wurden 23,5 g (0,42 mol) Kaliumhydroxid (Firma J.T. Baker, 100 %ig) in 23,5 g Wasser gelöst und unter Rühren in den Kolben gegeben und für 30 Minuten bei Raumtemperatur gerührt. Daraufhin wurde die Lösung auf 80°C erwärmt und 422,3 g (0,42 mol) Cetyltrimethylammoniumchlorid (Firma Evonik Industries AG, 30 % in Wasser) hinzugegeben und für weitere zwei Stunden bei 80°C und weiterhin über Nacht bei Raumtemperatur gerührt. Das Wasser wurde unter Anlegen eines Vakuums bei 60°C abdestilliert. Das entstandene Produkt wurde in *tert*-Butanol (Firma Sigma Aldrich, >97,8 %) gelöst und mit wenig Natriumsulfat versetzt und der Niederschlag abfiltriert. Schließlich wurde das Lösungsmittel unter Anlegen eines Vakuums bei 60°C abdestilliert.

Das $^{13}$C-NMR bestätigte die Herstellung eines 1:1 Salzes aus Arginat und Cetyltrimethylammonium.

*Synthesebeispiel S2: Herstellung von Cetyltrimethylammoniumlysinat*

63,9 g (0,45 mol) Lysin (Firma ABCR, 97 %ig) wurden im 1 L-Vierhalskolben mit Innenthermometer, KPG-Rührer und Kugelkühler in Wasser suspendiert. Anschließend wurden 25,8 g (0,42 mol) Kaliumhydroxid (Firma J.T. Baker, 100 %ig), gelöst in 25,8 g Wasser, unter Rühren in den Kolben gegeben und für für 30 Minuten bei Raumtemperatur gerührt. Daraufhin wurde die Lösung auf 80°C erwärmt und 460,2 g (0,45 mol) Cetyltrimethylammoniumchlorid (Firma Evonik Industries AG, 30 % in Wasser) hinzugegeben und für zwei weitere Stunden bei 80°Cund dann über Nacht bei

Raumtemperatur gerührt. Das Wasser wurde unter Anlegen eines Vakuums bei 60°C abdestilliert. Das entstandene Produkt wurde in tert-Butanol (Firma Sigma Aldrich, >97,8 %) gelöst und mit wenig Natriumsulfat versetzt und der sich gebildete Niederschlag abfiltriert. Schließlich wurde das Lösungsmittel unter Anlegen eines Vakuums bei 60°C abdestilliert.

Das $^{13}$C-NMR bestätigte die Herstellung eines 1:1 Salzes aus Lysinat und Cetyltrimethylammonium.

*Synthesebeispiel S3: Herstellung von Behenyltrimethylammoniumlysinat*

48 g (0,32 mol) Lysin (Firma ABCR, 97 %ig) wurden im 0,5L-Vierhalskolben mit Innenthermometer, KPG-Rührer und Kugelkühler in 56 g Ethanol suspendiert. Anschließend wurden 17,9 g (0,32 mol) Kaliumhydroxid (Firma J.T. Baker, 100 %) in 40 g Ethanol gelöst und unter Rühren in den Reaktionskolben gegeben und für 30 Minuten bei Raumtemperatur gerührt. Daraufhin wurde die Lösung auf 80°C erwärmt und 150 g (0,32 mol) Behenyltrimethylammoniumchlorid (Firma Evonik Industries AG, 85% in Isopropanol) gelöst in 150 g Isopropanol, hinzugegeben. Nach zwei Stunden bei 80°C wurde die Lösung über Nacht bei Raumtemperatur gerührt. Der entstandene Niederschlag wurde mittels einer einfachen Filtration abgetrennt, und das Lösungsmittel unter Anlegen eines Vakuums bei 60°C abdestilliert.

Das $^{13}$C-NMR bestätigte die Herstellung eines 1:1 Salzes aus Lysin und Behenyltrimethylammonium.

*Synthesebeispiel S4: Umsetzung von α,ω-Epoxysiloxan mit N=80 mit Behenyltrimethylammoniumlysinat*

100 g (0,018 mol) α,ω-Epoxysiloxan (N=80, M=5517,2 g/mol, Epoxywert: 0,58%, Firma Evonik Industries AG), 18,5 g (0,036 mol) Behenyltrimethylammoniumlysinat aus Synthesebeispiel S3 und 29,7 g *tert*-Butanol (Firma Sigma Aldrich, >97,8 %) wurden im 0,25 L-Vierhalskolben mit Innenthermometer, Kugelkühler und KPG-Rührer vorgelegt. Die Mischung wurde für fünf Stunden bei 85°C gerührt. Anschließend wurde das Lösungsmittel unter Anlegen eines Vakuums bei 60°C abdestilliert.

$^{1}$H-NMR und GPC bestätigten die Herstellung des aminosäuremodifizierten Siloxans.

*Synthesebeispiel S5: Umsetzung von α,ω-Epoxysiloxan mit N=80 und Cetyltrimethylammoniumlysinat*

100 g (0,018 mol) α,ω-Epoxysiloxan (N=80, M=5517,2 g/mol, Epoxywert: 0,58%, Firma Evonik Industries AG), 15,5 g (0,036 mol) Cetyltrimethylammoniumlysinat aus Synthesebeispiel S2 und 28,9 g *tert*-Butanol (Firma Sigma Aldrich,>97,8 %) wurden im 0,25 L-Vierhalskolben mit Innenthermometer, Kugelkühler und KPG-Rührer vorgelegt und auf 85°C erwärmt. Diese Mischung wurde für fünf Stunden gerührt, woraufhin das Lösungsmittel unter Anlegen eines Vakuums bei 60°C abdestilliert wurde.

$^{1}$H-NMR und GPC bestätigten die Herstellung des aminosäuremodifizierten Siloxans.

*Synthesebeispiel S6: Umsetzung von α,ω-Epoxysiloxan N=80 und Cetyltrimethylammoniumarginat*

100 g (0,018mol) α,ω-Epoxysiloxan (N=80, M=5517,2g/mol, Epoxywert: 0,58%, Firma Evonik Industries AG), 15,9 g (0,036mol) Cetyltrimethylammoniumarginat aus Synthesebeispiel S1 und 29 g *tert*-Butanol (Firma Sigma Aldrich, >97,8 %) wurden im 0,25 L-Vierhalskolben mit Innenthermometer, Kugelkühler und KPG-Rührer auf 85°C erwärmt und für vier Stunden bei dieser Temperatur gerührt. Anschließend wurde das Lösungsmittel unter Anlegen eines Vakuums bei 60°C abdestilliert.

$^{1}$H-NMR und GPC bestätigten die Herstellung des aminosäuremodifizierten Siloxans.

*Synthesebeispiel S7: Umsetzung eines T-strukturellen Epoxysiloxans mit N=150 und Cetyltrimethylammoniumarginat*

50 g (0,0047 mol) eines Epoxysiloxans (N=150, M=10666 g/mol, Epoxywert: 0,45%, Firma Evonik Industries AG), 6,2 g (0,014 mol) Cetyltrimethylammoniumarginat aus Synthesebeispiel S1 und 28 g *tert*-Butanol (Firma Sigma Aldrich, >97,8 %) wurden im 0,25 L-Vierhalskolben mit Innenthermometer, Kugelkühler und KPG-Rührer vorgelegt, auf 85°C erwärmt und für vier Stunden gerührt. Schließlich wurde das Lösungsmittel unter Anlegen eines Vakuums 60°C abdestilliert.

$^{1}$H-NMR und GPC bestätigten die Herstellung des aminosäuremodifizierten Siloxans.

*Synthesebeispiel S8: Umsetzung eines α,ω-Epoxysiloxans mit N=30 und Cetyltrimethylammoniumarginat*

50 g (0,021mol) eines α,ω-Epoxysiloxans(N=30, M=2335,8 g/mol, Epoxywert: 1,37%, Firma Evonik Industries AG), 18,8 g (0,042 mol) Cetyltrimethylammoniumarginat aus Synthesebeispiel S1 und 17,2 g *tert*-Butanol (Firma Sigma Aldrich, >97,8 %) wurden im 0,25 L-Vierhalskolben mit Innenthermometer, Kugelkühler und KPG-Rührer vorgelegt. Die

Mischung wurde auf 85°C erwärmt und für vier Stunden bei dieser Temperatur gerührt. Anschließend wurde das Lösungsmittel durch Anlegen eines Vakuums bei 60°C abdestilliert.
$^1$H-NMR und GPC bestätigten die Herstellung des aminosäuremodifizierten Siloxans.

*Synthesebeispiel S9: Herstellung und Umsetzung eines Tetraepoxytetramethyltetracyclosiloxan mit Cethyltrimethylammoniumarginat*

123,66 g (1,08mol) Allylglycidylether (Firma Sigma Aldrich) werden in 123,66 g Toluol vorgelegt und auf 80°C erwärmt. 0,17g Karstedt-Kat (1 %ige Lösung in Cyclen, 10 ppm Platin bezogen auf den Ansatz) werden dann hinzugegeben. 50 g (0,83 mol) Methylhydrocyclosiloxan (ABCR, 92 %) in 25g Toluol werden langsam zugetropft. Regelmäßige volumetrische Si-H Umsatz Bestimmung bis 100% Umsatz. Anschließend wird überschüssiges Toluol und Allylglycidylether am Rotationsverdampfer bei 100 °C und Vakuum abdestilliert.

55,2 g (0,121 mol) Cethyltrimethylammoniumarginat aus Synthesebeispiel 1 und 55,2 g *tert*-Butanol (Firma Sigma Aldrich, >97,8 %) werden in 0,25 L Vierhalskolben mit Kugelkühler und Tropftrichter vorgelegt und auf 82°C erwärmt. 21 g (0,121 mol) des Umsetzungsproduktes aus Allylglycidylether und Methylhydrocyclosiloxan werden in 21 g *tert*-Butanol langsam zugetropft und für weitere 2 Stunden gerührt. *Tert*-Butanol wird schließlich bei 80°C am Rotationsverdampfer abdestilliert.
$^1$H-NMR bestätigte die Herstellung des aminosäuremodifizierten Siloxans.

*Synthesebeispiel S10: Herstellung von Cetyltrimethylammoniumhistidat*

57,8 g (0,365mol) Histidin (Firma ABCR, 98 %) werden im 1 L-Vierhalskolben mit Innenthermometer, KPG-Rührer und Kugelkühler in Wasser suspendiert. 20,5 g (0,365mol) Kaliumhydroxid (Firma J.T. Baker, 100 %), gelöst in 20 g Wasser, werden unter Rühren in den Kolben gegeben und für 30 Minuten bei Raumtemperatur gerührt. Anschließend wird die Lösung auf 80°C erwärmt und 367,5 g (0,344 mol) Cethyltrimethylammoniumchlorid (Firma Evonik Industries AG, 30%ig in Wasser) hinzugegeben und für weitere zwei Stunden bei 80°C und schließlich über Nacht bei Raumtemperatur gerührt. Unter Anlegen eines Vakuums bei 60°C wird das Wasser abdestilliert. Das entstandene Aminosäuresalz wird in *tert*-Butanol (Firma Sigma Aldrich, >97,8 %) und Natriumsulfat aufgenommen und der entstehende Niederschlag abfiltriert. Das reine Salz wird dann durch Destillation des *tert*-Butanol erhalten.

Das $^{13}$C-NMR bestätigte die Herstellung eines 1:1 Salzes aus Histidat und Cetyltrimethylammonium.

*Synthesebeispiel S11: Herstellung von Cetyltrimethylammoniumglutamat*

73,5 g (0,498 mol) Glutamin (Firma Sigma Aldrich, 99 %) werden im 1 L-Vierhalskolben mit Innenthermometer, KPG-Rührer und Kugelkühler in Wasser suspendiert. 27,9 g (0,498mol) Kaliumhydroxid (Firma J.T. Baker, 100%ig), gelöst in 27,9 g Wasser, werden dann unter Rühren in den Kolben gegeben und 30 Minuten gerührt bevor die Lösung auf 80°C erwärmt wird und 500 g (0,468 mol) Cethyltrimethylammoniumchlorid (Firma Evonik Industries AG, 30 % in Wasser) zugegeben werden. Die Lösung wird für weitere zwei Stunden bei 80°C und dann über Nacht bei Raumtemperatur gerührt. Das entstandene Aminosäuresalz wird in tert-Butanol (Firma Sigma Aldrich, >97,8 %) und Natriumsulfat aufgenommen und der entstehende Niederschlag abfiltriert. Das reine Salz wird dann durch Destillation des *tert*-Butanol erhalten.

Das $^{13}$C-NMR bestätigte die Herstellung eines 1:1 Salzes aus Glutamat und Cetyltrimethylammonium.

*Synthesebeispiel S12: Herstellung von Trihexyltetradecylphosphoniumlysinat*

6,9 g (0,046 mol) Lysin (Firma ABCR, 97 %) werden im 0,25L-Vierhalskolben mit Innenthermometer, KPG-Rührer und Kugelkühler in 20 g Ethanol suspendiert bevor 2,6 g (0,046 mol) Kaliumhydroxid (Firma J.T. Baker, 100 %), gelöst in 10 g Ethanol, unter Rühren zugegeben werden und für 30 Minuten gerührt. Anschließend wird die Lösung auf 80°C erwärmt und 25 g (0,046 mol) Trihexyltetradecylphosphoniumchlorid (Firma Sigma Aldrich, 95 %) gelöst in 40 g Isopropanol, hinzugegeben. Die Lösung wird für weitere zwei Stunden bei 80°C und dann über Nacht bei Raumtemperatur gerührt. Der entstandene Niederschlag wird abfiltriert und das das Lösungsmittel im Filtrat bei 60°C unter Anlegen eines Vakuums abdestilliert.

Das $^{13}$C-NMR bestätigte die Herstellung eines 1:1 Salzes aus Lysinat und Trihexyltetradecylphosphonium.

*Synthesebeispiel S13: Herstellung von 1-Methyl-3-octylimidazoliumlysinat*

15,8 g (0,105mol) Lysin (Firma ABCR, 97 %) werden im 0,25 L-Vierhalskolben mit Innenthermometer, KPG-Rührer und Kugelkühler in 30 g Ethanol suspendiert bevor 5,8 g (0,105mol) Kaliumhydroxid (Firma J.T. Baker, 100 %),

gelöst in 10 g Ethanol, unter Rühren zugegeben werden und für 30 Minuten gerührt. Anschließend wird die Lösung auf 80°C erwärmt und 25 g (0,105 mol) 1-Methyl-3-octylimidazolimchlorid (Firma Sigma Aldrich, 97 %), gelöst in 30 g Isopropanol, hinzugegeben. Die Lösung wird für weitere zwei Stunden bei 80°C und dann über Nacht bei Raumtemperatur gerührt. Der entstandene Niederschlag wird abfiltriert und das das Lösungsmittel im Filtrat bei 60°C unter Anlegen eines Vakuums abdestilliert.

Das $^{13}$C-NMR bestätigte die Herstellung eines 1:1 Salzes aus Lysinat und 1-Methyl-3-octylimidazolium.

*Synthesebeispiel S14: Umsetzung von α,ω-carbonathaltigem Siloxan mit N= 80 und Cethyltrimethylammoniumlysinat*

6,9 g (0,016 mol) Cethyltrimethylammoniumlysinat aus Synthesebeispiel S2 und 14,3 g *tert*-Butanol (Firma Sigma Aldrich, >97,8 %) wurden im 0,25 L-Vierhalskolben mit Innenthermometer, Kugelkühler und KPG-Rührer vorgelegt, auf 85°C erwärmt und 50 g (0,008 mol) eines α,ω-carbonathaltigem Siloxan mit N= 80 (M=6224 g/mol) hergestellt wie beschrieben in US5606077 innerhalb von 60 Minuten zugetropft. Die Reaktion wurde für weitere neun Stunden gerührt. Schließlich wurde das Lösungsmittel *tert*-Butanol bei 60°C am Rotationsverdampfer abdestilliert.

Das $^{1}$H-NMR und das $^{13}$C-NMR bestätigten die erfolgreiche Umsetzung zum aminosäuretragenden Siloxan.

*Synthesebeispiel S15: Umsetzung von α,ω-Epoxysiloxan N=80 und monoepoxypolyether mit Cetyltrimethylammoniumarginat*

100 g (0,018mol) α,ω-Epoxysiloxan (N=80, M=5517,2g/mol, Epoxywert: 0,58%, Firma Evonik Industries AG), 15,9 g (0,036mol) Cetyltrimethylammoniumarginat aus Synthesebeispiel S1 und 29 g *tert*-Butanol (Firma Sigma Aldrich, >97,8 %) wurden im 0,25 L-Vierhalskolben mit Innenthermometer, Kugelkühler und KPG-Rührer auf 85°C erwärmt und für vier Stunden bei dieser Temperatur gerührt. Anschließend wird ein monoepoxypolyether (ipox chemicals, epoxy quivalent: 470-500) zugegeben und für weitere 2 Stunden bei 80 °C gerührt. Schließlich wird das Lösungsmittel unter Anlegen eines Vakuums bei 60°C abdestilliert.

*Anwendungsbeispiele Textilpflege*

Dem Fachmann ist bekannt, dass die Aufbringung von Textilhilfsmitteln wie zum Beispiel Weichspülern zu einer Oberflächenhydrophobierung führen kann. Diese Oberflächenhydrophobierung zeigt sich in einem schlechteren Rücknetzverhalten des Textils gegenüber Wasser. Die erfindungsgemäßen Copolymere bewirken eine deutliche Hydrophilierung von Textilfasern und vermögen sogar in Kombination mit sich negativ auf das Rücknetzverhalten auswirkenden Textilhilfsmitteln eine deutliche Reduktion der durch diese hervorgerufene Hydrophobierung.

Zur Überprüfung der bei der Anwendung von erfindungsgemäßen aminosäurehaltigen Siloxanen auf textilen Fasern erreichbaren Hydrophilie, wurden verschiedene Normtextilien ausgerüstet und exemplarisch gegen einen kommerziell erhältlichen Weichspülerwirkstoff (Rewoquat WE18, Evonik Industries) hinsichtlich seines Rücknetzvermögens mittels eines Steighöhentests abgeprüft.

Verwendete Normtextilien:

Baumwollgewebe wfl Code 13A, Polyestergewebe wfk Code 30A, beide erhältlich bei der wfk Testgewebe GmbH, Krefeld.

Ausrüstung der Textilprüfkörper (Baumwolle) durch Zwangsapplikation

Aus einer Stoffbahn wurden 25 x 7,5 cm große Textilprüfkörper herausgetrennt, die eine Masse von 4 g aufwiesen. Es wurden sodann in 1L PE-Kunststoffflaschen Ausrüstungsflotten bestehend aus den jeweiligen Wirkstoffen (0,077 Gew.-%) in Wasser (16° dH) angesetzt. Die so hergestellten Flotten wurden für 2 Stunden gerührt um eine homogene Aufnahme des Wirkstoffes zu gewährleisten. Die einzelnen Textilprüfkörper wurden sodann in einen Teflonbecher mit 13,7 g der zuvor hergestellten Flottenlösung benetzt und mittels eines Polyethylen-Spatels in der Flotte bewegt. Nach exakt 10 Minuten wurden die Prüfkörper aus der Flotte herausgenommen, an einer Leine zur Trocknung befestigt und erst am Folgetag vermessen.

Ausrüstung der Textilprüfkörper (Polyester) durch Zwangsapplikation:

Aus einer Stoffbahn wurden 25 x 7,5 cm große Textilprüfkörper herausgetrennt, die eine Masse von 3 g aufwiesen. Es wurden sodann in 1L PE-Kunststoffflaschen Ausrüstungsflotten bestehend aus den jeweiligen Wirkstoffen (0,077 Gew.-%) in Wasser (16° dH) angesetzt. Die so hergestellten Flotten wurden für 2 Stunden gerührt um eine

homogene Aufnahme des Wirkstoffes zu gewährleisten. Die einzelnen Textilprüfkörper wurden sodann in einen Teflonbecher mit 10,3 g der zuvor hergestellten Flottenlösung benetzt und mittels eines Polyethylen-Spatels in der Flotte bewegt. Nach exakt 10 Minuten wurden die Prüfkörper aus der Flotte herausgenommen, an einer Leine zur Trocknung befestigt und erst am Folgetag vermessen.

Prüfung der Hydrophilie:

Zur Überprüfung der Hydrophilie wurde eine an DIN 53924 angelehnte Prüfmethode zur Messung der Steighöhe von Wasser verwendet. Die Messungen werden dabei immer zu einer Vergleichsprobe durchgeführt und aus diesem Grund wurd auf eine klimatisierte Umgebung verzichtet. Es wurd immer nur innerhalb einer Messreihe verglichen. Dabei wurd das ausgerüstete Testgewebe in jeweils fünf 25 cm lange und 1,5 cm breite Streifen geschnitten, mit einem wasserlöslichen Stift seitlich markiert und an einer Halterung senkrecht straff aber ohne Spannung befestigt. Die Halterung wird anschließend für fünf Minuten so in ein Wasserbecken gestellt, sodass 2 cm der Streifen ins Wasser eintauchen. Die wasserlösliche Markierung dient der besseren Erkennbarkeit der Steighöhe durch das Verlaufen der Farbe bei Benetzung mit Wasser. Nachdem die Halterung 10 Minuten außerhalb des Wasserbeckens gestanden hat, wird die Steighöhe in cm abgelesen und gegen den Blindwert (Steighöhe der unbehandelten Baumwollstreifen x cm = 100 %) bestimmt und in % vom Blindwert angegeben.
Die Ergebnisse sind in den folgenden beiden Tabellen angegeben.

Bestimmung der Steighöhe auf Baumwollwebware.

| Zusammensetzung | Steighöhe in % des Blindwertes |
|---|---|
| Vergleichsbeispiel Rewoquat® WE-18 | 63,0 |
| Synthesebeispiel S8 | 86,0 |
| Synthesebeispiel S8 + Rewoquat® WE-18 (20:80) | 72,0 |
| Synthesebeispiel S6 - 182 | 88,0 |
| Synthesebeispiel S6-182 + Rewoquat® WE-18 (20:80) | 71,0 |
| Unbehandelt | 100,0 |

Es zeigt sich bei Applikation auf ein Baumwolltextil eine deutlich gesteigerte Hydrophilie der Faser gegenüber dem Vergleichsbeispiel. Auch bewirken schon geringe Zugaben von nur 20 % an der Gesamtwirkstoffmenge eine deutliche Minderung der Hydrophobierung durch den Weichspülerwirkstoff. Beides ist im Besonderen daher überraschend, da dem Fachmann Siloxane als stark hydrophobierend bekannt sind.

Bestimmung der Steighöhe auf Polyestergewebe.

| Zusammensetzung | Steighöhe in % des Blindwertes |
|---|---|
| Vergleichsbeispiel Rewoquat® WE-18 | 65,0 |
| Synthesebeispiel S8 | 97,0 |
| Synthesebeispiel S8 + Rewoquat® WE-18 (20:80) | 95,0 |
| Synthesebeispiel S6 | 90,0 |
| Synthesebeispiel S6 + Rewoquat® WE-18 (20:80) | 88,0 |
| Unbehandelt | 100,0 |

Weiterhin hat sich gezeigt, dass bei Applikation auf Polyestergewebe eine deutlich gesteigerte Hydrophilie der Faser gegenüber dem Vergleichsbeispiel, die fast an den Blindwert hinreicht, resultiert. Auch bewirken schon geringe Zugaben von nur 20 % an der Gesamtwirkstoffmenge eine deutliche Minderung der Hydrophobierung durch den Weichspülerwirkstoff. Beides ist im Besonderen daher überraschend, da dem Fachmann Siloxane als stark hydrophobierend bekannt sind.

*Anwendungsbeispiele Haarpflege:*

*1.) Austestung der Konditionierung von Haar mittels Sensoriktests in einer Haarspülung:*

Für die anwendungstechnische Beurteilung der Konditionierung von Haar wurden die erfindungsgemäßen Verbindungen aus den Synthesebeispielen S8, S6 und S5 sowie das kommerziell erhältliche Produkt ABIL® Quat 3272

(INCl: Quaternium-80, Hersteller Evonik Industries) in einer einfachen kosmetischen Haarspülungs-Formulierung eingesetzt.

Die anwendungstechnischen Eigenschaften beim Einsatz in Haarspülungen wurden in den folgenden Rezepturen überprüft:

| Formulierungsbeispiele | 0a | 1a | 2a | 3a | V4a |
|---|---|---|---|---|---|
| TEGINACID® C, Evonik Industries (INCI: Ceteareth-25) | 0,5% | 0,5% | 0,5% | 0,5% | 0,5% |
| TEGO® Alkanol 16, Evonik Industries (INCI: Cetyl Alcohol) | 5,0% | 5,0% | 5,0% | 5,0% | 5,0% |
| VARISOFT® 300, 30%-ig, Evonik Industries (INCI: Cetrimonium Chloride (=CTAC)) | 2,0% | 2,0% | 2,0% | 2,0% | 2,0% |
| Neolone PE, The Dow Chemical Company (INCI: Phenoxyethanol; Methylisothiazolinone) | 0,45% | 0,45% | 0,45% | 0,45% | 0,45% |
| Wasser, demineralisiert | ad. 100,0% | | | | |
| Zitronensäure | ad. pH 4,5 ± 0,3 | | | | |
| Synthesebeispiel S8, 80%ig in Isopropanol (erfindungsgemäß) | | 0,38% | | | |
| Synthesebeispiel S6, 80%ig in Isopropanol (erfindungsgemäß) | | | 0,38% | | |
| Synthesebeispiel S5, 80%ig in Isopropanol (erfindungsgemäß) | | | | 0,38% | |
| ABIL® Quat 3272, 50%ig in Propylenglycol (nicht erfindungsgemäß) | | | | | 0,60% |

Für die anwendungstechnische Beurteilung wurden Haartressen, die für sensorische Tests verwendet werden, durch eine Bleichbehandlung standardisiert vorgeschädigt. Dazu wurden friseurübliche Produkte verwendet. Der Testablauf, die verwendeten Basismaterialien sowie die Details der Beurteilungskriterien wurden in DE 103 27 871 beschrieben.

Die Vorbehandlung der Haare erfolgte durch ein Shampoo, welches keine Konditioniermittel enthält.

Standardisierte Behandlung von vorgeschädigten Haartressen mit konditionierenden Formulierungen:

Die, wie oben beschrieben, vorgeschädigten Haartressen wurden wie folgt mit der oben beschriebenen konditionierenden Spülung behandelt:

> Die Haartressen wurden unter fließendem, warmem Wasser benetzt. Das überschüssige Wasser wurde leicht von Hand ausgedrückt, dann wird das Shampoo aufgebracht und sanft im Haar eingearbeitet (1 ml/Haartresse (2 g)). Nach einer Verweilzeit von 1 min wurde das Haar für 1 min gespült. Direkt im Anschluss wurde die Spülung aufgebracht und sanft im Haar eingearbeitet (1 ml/Haartresse (2 g)). Nach einer Verweilzeit von 1 min wurde das Haar für 1 min gespült.

Beurteilungskriterien:

Die sensorischen Bewertungen erfolgten nach Noten, die auf einer Skala von 1 bis 5 vergeben wurden, wobei 1 die schlechteste und 5 die beste Bewertung ist. Die einzelnen Testkriterien erhielten jeweils eine eigene Bewertung. Die Testkriterien sind: Nasskämmbarkeit, Nassgriff, Trockenkämmbarkeit, Trockengriff, Aussehen/Glanz.

In der folgenden Tabelle wurden die Ergebnisse der sensorischen Beurteilung der wie oben beschrieben durchgeführten Behandlung der Haarsträhnchen mit den erfindungsgemäßen Formulierungen 1a, 2a und 3a, der Vergleichsformulierung V4a und der Kontrollformulierung 0a (Placebo ohne Testsubstanz) verglichen.

| | Nasskämmbarkeit | Nassgriff | Trockenkämmbarkeit | Trockengriff | Glanz |
|---|---|---|---|---|---|
| Kontrollformulierung 0a | 3,9 | 4,0 | 4,1 | 4,5 | 3,0 |
| Erfindungsgemäße Formulierung 1a | 4,5 | 4,1 | 4,3 | 4,4 | 4,0 |

(fortgesetzt)

| | Nasskämmbarkeit | Nassgriff | Trockenkämmbarkeit | Trockengriff | Glanz |
|---|---|---|---|---|---|
| Erfindungsgemäße Formulierung 2a | 4,9 | 4,4 | 4,5 | 4,7 | 4,5 |
| Erfindungsgemäße Formulierung 3a | 4,9 | 4,5 | 4,8 | 4,8 | 4,5 |
| Vergleichsformulierung (nicht erfindungsgemäß) V4a | 4,7 | 4,3 | 4,6 | 4,8 | 3,5 |

Die erfindungsgemäßen Formulierungen 1a, 2a und 3a mit den erfindungsgemäßen Verbindungen aus den Synthesebeispielen S8, S6 und S5 zeigten in der sensorischen Beurteilung gute kosmetische Bewertungen. Die Kontrollformulierung 0a (mit CTAC) wurde durch die Zugabe von lediglich 0.3% aktiv Siliconprodukt signifikant verbessert. Dabei wurden die bereits sehr guten Eigenschaften der Vergleichsformulierung V4a bzgl. Nasskämmbarkeit und Nassgriff durch die erfindungsgemäßen Formulierungen 2a und 3a mit den erfindungsgemäßen Verbindungen Bespiel 2 und 3 noch weiter gesteigert. Eine signifikant bessere Bewertung wurde auch beim Glanz durch die Verwendung der erfindungsgemäßen Formulierungen 1a, 2a und 3a erreicht.

*2.) Austestung der Konditionierung von Haar mittels Sensoriktests aus einem Shampoo:*

Neben der o.g. Haarspülungsformulierung wurden für die anwendungstechnische Beurteilung der Konditionierung von Haar die erfindungsgemäßen Verbindungen aus den Synthesebeispielen S8, S6 und S5 sowie das kommerziell erhältliche Produkt ABIL® Quat 3272 (INCI: Quaternium-80, Hersteller Evonik Industries) auch in einer einfachen kosmetischen Shampoo-Formulierung eingesetzt.

Die anwendungstechnischen Eigenschaften beim Einsatz in Shampoos wurden in den folgenden Rezepturen überprüft:

| Formulierungsbeispiele | 0b | 1b | 2b | 3b | V4b |
|---|---|---|---|---|---|
| Texapon NSO-IS, 28%-ig, BASF (INCI: Sodium Laureth Sulfate) | 32,0% | 32,0% | 32,0% | 32,0% | 32,0% |
| TEGO® Betain F 50, 38%-ig, Evonik Industries (INCI: Cocamidopropyl Betaine) | 8,0% | 8,0% | 8,0% | 8,0% | 8,0% |
| Polymer JR 400, Amerchol (INCI: Polyquaternium-10 (=PQ-10)) | 0,2% | 0,2% | 0,2% | 0,2% | 0,2% |
| ANTIL® 171, Evonik Industries (INCI: PEG-18 Glyceryl Oleate/Cocoate) | 2,5% | 2,5% | 2,5% | 2,5% | 2,5% |
| NaCl | 0,5% | 0,5% | 0,5% | 0,5% | 0,5% |
| Neolone PE, The Dow Chemical Company (INCI: Phenoxyethanol; Methylisothiazolinone)) | 0,45% | 0,45% | 0,45% | 0,45% | 0,45% |
| Wasser, demineralisiert | ad. 100,0% | | | | |
| Zitronensäure | ad. pH 5,5 ± 0,3 | | | | |
| Synthesebeispiel S8, 80%ig in Isopropanol (erfindungsgemäß,) | | 1,25% | | | |
| Synthesebeispiel S6, 80%ig in Isopropanol (erfindungsgemäß) | | | 1,25% | | |
| Synthesebeispiel S5, 80%ig in Isopropanol (erfindungsgemäß) | | | | 1,25% | |
| ABIL® Quat 3272, 50%ig in Propylenglycol (nicht erfindungsgemäß) | | | | | 2,0% |
| Viskosität [mPa·s] | 3700 | 290 | 420 | 6000 | 150 |

Überraschenderweise sind selbst die Produktbeispiele 2 und 3 mit einer langen Siliconkette klar in dieser Shampooformulierung löslich.

Zudem ist überraschend, dass die erfindungsgemäßen Produkte nicht zu einer so starken Erniedrigung der

Viskositäten in dieser Shampoformulierung wie das Vergleichsprodukt führen. Besonders überraschend ist, dass die Verbindung aus Synthesebeispiel S5 sogar eine Erhöhung der Viskosität zeigt. Dieser Effekt ist von großer Relevanz für Formulierer, da der Einsatz von Verdickungsmitteln reduziert werden kann.

Für die anwendungstechnische Beurteilung wurden Haartressen wie oben unter 1.) beschrieben vorgeschädigt. Die Behandlung mit den konditionierenden ShampooFormulierungen erfolgte ebenfalls entsprechend dem oben beschriebenen Verfahren. Statt der Haarspülung wurden aber die Shampooformulierungen angewendet.

In der folgenden Tabelle wurden die Ergebnisse der sensorischen Beurteilung der wie oben beschrieben durchgeführten Behandlung der Haarsträhnchen mit den erfindungsgemäßen Formulierungen 1b, 2b und 3b, der Vergleichsformulierung V4b und der Kontrollformulierung 0b (Placebo ohne Testsubstanz) verglichen.

| | Nasskämmbarkeit | Nassgriff | Trockenkämmbarkeit | Trockengriff | Glanz |
|---|---|---|---|---|---|
| Kontrollformulierung 0b | 2,9 | 2,9 | 3,9 | 4,1 | 3,0 |
| Erfindungsgemäße Formulierung 1 b | 4,3 | 4,0 | 4,0 | 4,0 | 4,0 |
| Erfindungsgemäße Formulierung 2b | 4,4 | 4,1 | 4,7 | 4,5 | 4,5 |
| Erfindungsgemäße Formulierung 3b | 4,1 | 4,5 | 4,8 | 4,8 | 4,0 |
| Vergleichsformulierung (nicht erfindungsgemäß) V4b | 4,0 | 3,8 | 4,1 | 4,4 | 3,5 |

Die erfindungsgemäßen Formulierungen 1b, 2b und 3b mit den erfindungsgemäßen Verbindungen aus den Synthesebeispielen S8, S6 und S5 zeigten in der sensorischen Beurteilung sehr gute kosmetische Bewertungen. Die Kontrollformulierung 0b (mit PQ-10) wurde durch die Zugabe der Siliconprodukte signifikant verbessert. Speziell die erfindungsgemäßen Formulierungen 2b und 3b mit den erfindungsgemäßen Verbindungen aus den Synthesebeispielen S6 und S5 zeigten durchweg sogar sehr viel bessere Werte als die bereits gute Vergleichsformulierung V4b. Überraschend sind hier speziell die äußerst guten Werte bzgl. Trockenkämmbarkeit und Trockengriff, da eine Differenzierung am trockenen Haar normalerweise schwierig ist. Bei Nasskämmbarkeit und Nassgriff war auch die erfindungsgemäße Formulierung 1 b mit der erfindungsgemäßen Verbindung Bespiel 1 signifikant besser als die Vergleichsformulierung V4b. Auch beim Glanz zeigte sich die Überlegenheit der erfindungsgemäßen Formulierungen 1b, 2b und 3b.

*Weitere Formulierungsbeispiele:*

Die in den nachfolgenden Tabellen angegebenen Formulierungsbeispiele zeigen exemplarische Vertreter einer Vielzahl von möglichen erfindungsgemäßen Zusammensetzungen.

Falls die Herstellung der Formulierung zuvor die getrennte Zubereitung bzw. Mischung von Formulierungsbestandteilen erfordert, wird dieses als mehrphasige Zubereitung bezeichnet.

Falls eine zweiphasige Herstellung erforderlich ist, werden die beiden Phasen mit A und B in den angegebenen Tabellen gekennzeichnet. Bei dreiphasigen Prozessen werden die drei Phasen mit A, B und C benannt. Wenn nicht anders angegeben handelt es sich bei den Angaben in den folgenden Tabellen um Angaben in Gew.-%.

Formulierungsbeispiel 1) Clear Shampoo

| | |
|---|---|
| TEXAPON® NSO, BASF, 28%-ig (INCI: Sodium Laureth Sulfate) | 32,00% |
| Synthesebeispielen S8 | 2,50% |
| Perfume | 0,50% |
| Water | 55,50% |
| TEGO® Betain F 50, Evonik Industries, 38%-ig (INCI: Cocamidopropyl Betaine) | 8,00% |
| ANTIL® 171, Evonik Industries (INCI: PEG-18 Glyceryl Oleate/Cocoate) | 1,00% |
| NaCl | 0,50% |
| Preservative | q.s. |

Formulierungsbeispiel 2) Shampoo, PEG- & sulfate-free

| | |
|---|---|
| REWOTERIC® AM C, Evonik Industries, 32%-ig (INCI: Sodium Cocoamphoacetate) | 15,00% |
| Plantapon ACG 50, BASF (INCI: Disodium Cocoyl Glutamate) | 3,80% |
| Synthesebeispielen S6 | 2,00% |
| Perfume | 0,30% |
| Water | 64,30% |
| TEGO® Betain F 50, Evonik Industries, 38%-ig (INCI: Cocamidopropyl Betaine) | 10,00% |
| VARISOFT® PATC, Evonik Industries (INCI: Palmitamidopropyltrimonium Chloride) | 2,30% |
| ANTIL® SPA 80, Evonik Industries (INCI: Isostearamide MIPA; Glyceryl Laurate) | 2,00% |
| Preservative | 0,30% |
| Citric Acid, 30 %-ig | q.s. |

Formulierungsbeispiel 3) Clear Conditioning Shampoo

| | |
|---|---|
| TEXAPON® NSO, BASF, 28%-ig (INCI: Sodium Laureth Sulfate) | 32,00% |
| ANTIL® 200, Evonik Industries (INCI: PEG-200 Hydrogenated Glyceryl Palmate; PEG-7 Glyceryl Cocoate) | 2,00% |
| Synthesebeispielen S5 | 2,00% |
| Perfume | 0,25% |
| Water | 55,25% |
| Polymer JR 400, Amerchol (INCI: Polyquaternium-10) | 0,20% |
| TEGO® Betain F 50, Evonik Industries, 38%-ig (INCI: Cocamidopropyl Betaine) | 8,00% |
| NaCl | 0,30% |
| Preservative | q.s. |

Formulierungsbeispiel 4) Clear Conditioning Shampoo

| | |
|---|---|
| TEXAPON® NSO, BASF, 28%-ig (INCI: Sodium Laureth Sulfate) | 32,00% |
| ANTIL® 200, Evonik Industries (INCI: PEG-200 Hydrogenated Glyceryl Palmate; PEG-7 Glyceryl Cocoate) | 2,00% |
| ABIL® Quat 3272, Evonik Industries (INCI: Quaternium-80) | 0,75% |
| Synthesebeispielen S8 | 1,50% |
| Perfume | 0,25% |
| Water | 55,00% |
| Polymer JR 400, Amerchol (INCI: Polyquaternium-10) | 0,20% |
| TEGO® Betain F 50, Evonik Industries, 38%-ig (INCI: Cocamidopropyl Betaine) | 8,00% |
| NaCl | 0,30% |
| Preservative | q.s. |

Formulierungsbeispiel 5) Clear Conditioning Shampoo

| | |
|---|---|
| TEXAPON® NSO, BASF, 28%-ig (INCI: Sodium Laureth Sulfate) | 32,00% |

(fortgesetzt)

| | |
|---|---|
| ANTIL® 200, Evonik Industries (INCI: PEG-200 Hydrogenated Glyceryl Palmate; PEG-7 Glyceryl Cocoate) | 2,00% |
| ABIL® B 8832, Evonik Industries (INCI: Bis-PEG/PPG-20/20 Dimethicone) | 0,50% |
| Synthesebeispielen S6 | 3,50% |
| Perfume | 0,25% |
| Water | 53,25% |
| Polymer JR 400, Amerchol (INCI: Polyquaternium-10) | 0,20% |
| TEGO® Betain F 50, Evonik Industries, 38%-ig (INCI: Cocamidopropyl Betaine) | 8,00% |
| NaCl | 0,30% |
| Preservative | q.s. |

Formulierungsbeispiel 6) Clear Conditioning Shampoo

| | |
|---|---|
| TEXAPON® NSO, BASF, 28%-ig (INCI: Sodium Laureth Sulfate) | 32,00% |
| VARISOFT® PATC, Evonik Industries (INCI: Palmitamidopropyltrimonium Chloride) | 1,50% |
| REWODERM® LI S 80, Evonik Industries (INCI: PEG-200 Hydrogenated Glyceryl Palmate; PEG-7 Glyceryl Cocoate) | 2,00% |
| Synthesebeispielen S8 | 2,50% |
| Perfume | 0,25% |
| Water | 52,05% |
| TEGO® Cosmo C 100, Evonik Industries (INCI: Creatine) | 1,00% |
| Jaguar C-162, Rhodia (INCI: Hydroxypropyl Guar Hydroxypropyltrimonium Chloride) | 0,20% |
| TEGO® Betain F 50, Evonik Industries, 38%-ig (INCI: Cocamidopropyl Betaine) | 8,00% |
| NaCl | 0,50% |
| Preservative | q.s. |

Formulierungsbeispiel 7) Clear Conditioning Shampoo

| | |
|---|---|
| TEXAPON® NSO, BASF, 28%-ig (INCI: Sodium Laureth Sulfate) | 32,00% |
| REWODERM® LI S 80, Evonik Industries (INCI: PEG-200 Hydrogenated Glyceryl Palmate; PEG-7 Glyceryl Cocoate) | 2,00% |
| Synthesebeispielen S6 | 2,50% |
| Perfume | 0,25% |
| Water | 53,55% |
| TEGO® Cosmo C 100, Evonik Industries (INCI: Creatine) | 1,00% |
| Jaguar C-162, Rhodia (INCI: Hydroxypropyl Guar Hydroxypropyltrimonium Chloride) | 0,20% |
| TEGO® Betain F 50, Evonik Industries, 38%-ig (INCI: Cocamidopropyl Betaine) | 8,00% |
| NaCl | 0,50% |
| Preservative | q.s. |

Formulierungsbeispiel 8) Pearlized Shampoo

| | |
|---|---|
| TEXAPON® NSO, BASF, 28%-ig (INCI: Sodium Laureth Sulfate) | 32,00% |
| Synthesebeispielen S8 | 5,50% |
| Perfume | 0,25% |
| Water | 49,25% |
| TEGO® Betain F 50, Evonik Industries, 38%-ig (INCI: Cocamidopropyl Betaine) | 8,00% |
| TEGO® Pearl N 300 Evonik Industries (INCI: Glycol Distearate; Laureth-4; Cocamidopropyl Betaine) | 2,00% |
| ANTIL® 171 Evonik Industries (INCI: PEG-18 Glyceryl Oleate/Cocoate) | 2,50% |
| NaCl | 0,50% |
| Preservative | q.s. |

Formulierungsbeispiel 9) Shampoo, PEG- & sulfate-free

| | | |
|---|---|---|
| A | REWOTERIC® AM C, Evonik Industries, 32%-ig (INCI: Sodium Cocoamphoacetate) | 20,00% |
| | REWOPOL® SB F 12 P, Evonik Goldschmidt, 96%-ig (INCI: Disodium Lauryl Sulfosuccinate) | 5,90% |
| | Synthesebeispielen S6 | 2,00% |
| | ANTIL® SPA 80, Evonik Industries, (INCI: Isostearamide MIPA; Glyceryl Laurate) | 1,70% |
| B | Water | 63,20% |
| | Citric Acid, 30%-ig | 3,60% |
| C | ANTIL® HS 60, Evonik Industries, (INCI: Cocamidopropyl Betaine; Glyceryl Laurate) | 3,00% |
| | Preservative | 0,60% |

Formulierungsbeispiel 10) Rinse-Off Conditioner

| | |
|---|---|
| Water | 85,50% |
| VARISOFT® BT 85, Evonik Industries (INCI: Behentrimonium Chloride) | 3,00% |
| Synthesebeispielen S6 | 5,50% |
| TEGO® Alkanol 1618, Evonik Industries (INCI: Cetearyl Alcohol) | 5,00% |
| Preservative, Perfume | q.s. |

Formulierungsbeispiel 11) Rinse-Off Conditioner

| | |
|---|---|
| Water | 90,20% |
| VARISOFT® EQ 65, Evonik Industries (INCI: Distearoylethyl Dimonium Chloride; Cetearyl Alcohol) | 2,00% |
| VARISOFT® BT 85, Evonik Industries (INCI: Behentrimonium Chloride) | 1,00% |
| Synthesebeispielen S8 | 1,80% |
| TEGO® Alkanol 1618, Evonik Industries (INCI: Cetearyl Alcohol) | 5,00% |
| Preservative, Perfume | q.s. |

Formulierungsbeispiel 12) Rinse-Off Conditioner

| | |
|---|---|
| Water | 87,20% |
| VARISOFT® EQ 65, Evonik Industries (INCI: Distearoylethyl Dimonium Chloride; Cetearyl Alcohol) | 2,00% |
| VARISOFT® BT 85, Evonik Industries (INCI: Behentrimonium Chloride) | 2,00% |
| ABIL® Quat 3272, Evonik Industries (INCI: Quaternium-80) | 0,50% |
| Synthesebeispielen S8 | 3,30% |
| TEGO® Alkanol 1618, Evonik Industries (INCI: Cetearyl Alcohol) | 5,00% |
| Preservative, Perfume | q.s. |

Formulierungsbeispiel 13) Rinse-Off Conditioner

| | |
|---|---|
| TEGINACID® C, Evonik Industries (INCI: Ceteareth-25) | 0,50% |
| TEGO® Alkanol 16, Evonik Industries (INCI: Cetyl Alcohol) | 2,00% |
| TEGO® Amid S 18, Evonik Industries (INCI: Stearamidopropyl Dimethylamine) | 1,00% |
| Synthesebeispielen S6 | 5,50% |
| Propylene Glycol | 2,00% |
| Citric Acid Monohydrate | 0,30% |
| Water | 88,70% |
| Preservative, Perfume | q.s. |

Formulierungsbeispiel 14) Rinse-Off Conditioner

| | |
|---|---|
| TEGINACID® C, Evonik Industries (INCI: Ceteareth-25) | 0,50% |
| TEGO® Alkanol 16, Evonik Industries (INCI: Cetyl Alcohol) | 5,00% |
| TEGOSOFT® DEC, Evonik Industries (INCI: Diethylhexyl Carbonate) | 1,00% |
| Synthesebeispielen S6 | 3,50% |
| Water | 87,20% |
| TEGO® Cosmo C 100 Evonik Industries (INCI: Creatine) | 0,50% |
| Propylene Glycol | 2,00% |
| Citric Acid Monohydrate | 0,30% |
| Preservative, Perfume | q.s. |

Formulierungsbeispiel 15) Leave-In Conditioner Spray

| | |
|---|---|
| Lactic Acid, 80% | 0,40% |
| Water | 92,30% |
| TEGO® Amid S 18, Evonik Industries (INCI: Stearamidopropyl Dimethylamine) | 1,20% |
| TEGIN® G 1100 Pellets, Evonik Industries (INCI: Glycol Distearate) | 0,60% |
| TEGO® Care PS, Evonik Industries (INCI: Methyl Glucose Sesquistearate) | 1,20% |
| TEGOSOFT® DEC, Evonik Industries (INCI: Diethylhexyl Carbonate) | 0,30% |
| Synthesebeispielen S8 | 4,00% |
| Preservative, Perfume | q.s. |

Formulierungsbeispiel 16) Leave-In Conditioner Spray

| | |
|---|---|
| TAGAT® CH 40, Evonik Industries (INCI: PEG-40 Hydrogenated Castor Oil) | 2,00% |
| Ceramide VI, Evonik Industries (INCI: Ceramide 6 II) | 0,05% |
| Perfume | 0,20% |
| Water | 81,95% |
| Synthesebeispielen S6 | 9,50% |
| LACTIL®, Evonik Industries (INCI: Sodium Lactate; Sodium PCA; Glycine; Fructose; Urea; Niacinamide; Inositol; Sodium benzoate; Lactic Acid) | 2,00% |
| TEGO® Betain F 50, Evonik Industries 38% (INCI: Cocamidopropyl Betaine) | 2,30% |
| Citric Acid (10% in water) | 2,00% |

Formulierungsbeispiel 17) Leave-In Conditioner Foam

| | |
|---|---|
| Synthesebeispielen S6 | 3,50% |
| TAGAT® CH 40, Evonik Industries (INCI: PEG-40 Hydrogenated Castor Oil) | 0,50% |
| Perfume | 0,30% |
| TEGO® Betain 810, Evonik Industries (INCI: Capryl/Capramidopropyl Betaine) | 2,00% |
| Water | 91,00% |
| TEGO® Cosmo C 100, Evonik Industries (INCI: Creatine) | 0,50% |
| TEGOCEL® HPM 50, Evonik Industries (INCI: Hydroxypropyl Methylcellulose) | 0,30% |
| VARISOFT® 300, Evonik Industries (INCI: Cetrimonium Chloride) | 1,30% |
| LACTIL® Evonik Industries (INCI: Sodium Lactate; Sodium PCA; Glycine; Fructose; Urea; Niacinamide; Inositol; Sodium benzoate; Lactic Acid) | 0,50% |
| Citric Acid (30% in water) | 0,10% |
| Preservative | q.s. |

Formulierungsbeispiel 18) Strong Hold Styling Gel

| | |
|---|---|
| TEGO® Carbomer 141, Evonik Industries (INCI: Carbomer) | 1,20% |
| Water | 65,00% |
| NaOH, 25% | 2,70% |
| PVP/VA W-735, ISP (INCI: PVP/VA Copolymer) | 16,00% |
| Synthesebeispielen S8 | 2,50% |
| Alcohol Denat. | 10,00% |
| TAGAT® O 2 V, Evonik Industries (INCI: PEG-20 Glyceryl Oleate) | 2,00% |
| Perfume | 0,30% |
| ABIL® B 88183, Evonik Industries (INCI: PEG/PPG-20/6 Dimethicone) | 0,30% |
| Preservative | q.s. |

Formulierungsbeispiel 19) Schaumiges Körperpflegemittel

| | |
|---|---|
| TEXAPON® NSO, BASF, 28%-ig (INCI: Sodium Laureth Sulfate) | 14,30% |

(fortgesetzt)

| | |
|---|---|
| Perfume | 0,30% |
| Synthesebeispielen S6 | 1,50% |
| REWOTERIC® AM C, Evonik Industries, 32%-ig (INCI: Sodium Cocoamphoacetate) | 8,00% |
| Water | 73,90% |
| TEGOCEL® HPM 50, Evonik Industries (INCI: Hydroxypropyl Methylcellulose) | 0,50% |
| LACTIL®, Evonik Industries (INCI: Sodium Lactate; Sodium PCA; Glycine; Fructose; Urea; Niacinamide; Inositol; Sodium benzoate; Lactic Acid) | 1,00% |
| Citric Acid Monohydrate | 0,50% |

Formulierungsbeispiel 20) Körperpflegemittel

| | |
|---|---|
| TEXAPON® NSO, BASF, 28%-ig (INCI: Sodium Laureth Sulfate) | 30,00% |
| TEGOSOFT® PC 31, Evonik Industries (INCI: Polyglyceryl-3 Caprate) | 0,50% |
| Synthesebeispielen S8 | 1,50% |
| Perfume | 0,30% |
| Water | 52,90% |
| TEGOCEL® HPM 4000, Evonik Industries (INCI: Hydroxypropyl Methylcellulose) | 0,30% |
| REWOTERIC® AM C, Evonik Industries, 32%-ig (INCI: Sodium Cocoamphoacetate) | 10,00% |
| Citric Acid Monohydrate | 0,50% |
| REWODERM® LI S 80, Evonik Industries (INCI: PEG-200 Hydrogenated Glyceryl Palmate; PEG-7 Glyceryl Cocoate) | 2,00% |
| TEGO® Pearl N 300, Evonik Industries (INCI: Glycol Distearate; Laureth-4; Cocamidopropyl Betaine) | 2,00% |

Formulierungsbeispiel 21) Schaumiges Körperpflegemittel

| | |
|---|---|
| TEXAPON® NSO, BASF, 28%-ig (INCI: Sodium Laureth Sulfate) | 14,30% |
| Perfume | 0,30% |
| Synthesebeispielen S6 | 1,00% |
| REWOTERIC® AM C, Evonik Industries, 32%-ig (INCI: Sodium Cocoamphoacetate) | 8,00% |
| Water | 75,10% |
| Polyquaternium-7, Nalco, (INCI: Merquat 550) | 0,30% |
| LACTIL®, Evonik Industries (INCI: Sodium Lactate; Sodium PCA; Glycine; Fructose; Urea; Niacinamide; Inositol; Sodium benzoate; Lactic Acid) | 0,50% |
| Citric Acid Monohydrate | 0,50% |

Formulierungsbeispiel 22) Mild Foam Bath

| | |
|---|---|
| TEXAPON® NSO, BASF, 28%-ig (INCI: Sodium Laureth Sulfate) | 27,00% |
| REWOPOL® SB FA 30, Evonik Industries, 40%-ig (INCI: Disodium Laureth Sulfosuccinate) | 12,00% |
| TEGOSOFT® LSE 65 K SOFT, Evonik Industries (INCI: Sucrose Cocoate) | 2,00% |
| Water | 38,00% |

(fortgesetzt)

| | |
|---|---|
| REWOTERIC® AM C, Evonik Industries, 32%-ig (INCI: Sodium Cocoamphoacetate) | 13,00% |
| Synthesebeispielen S8 | 1,50% |
| Citric Acid (30% in water) | 3,00% |
| ANTIL® 171, Evonik Industries (INCI: PEG-18 Glyceryl Oleate/Cocoate) | 1,50% |
| TEGO® Pearl N 300 Evonik Industries (INCI: Glycol Distearate; Laureth-4; Cocamidopropyl Betaine) | 2,00% |

Formulierungsbeispiel 23) Rinse-Off Conditioner

| | |
|---|---|
| Water | 88,20% |
| VARISOFT® 300, Evonik Industries (INCI: Cetrimonium Chloride) | 2,00% |
| VARISOFT® BT 85, Evonik Industries (INCI: Behentrimonium Chloride) | 2,00% |
| ABIL® OSW 5, Evonik Industries (INCI: Cyclopentasiloxane; Dimethiconol) | 1,00% |
| Synthesebeispielen S6 | 1,80% |
| TEGO® Alkanol 1618, Evonik Industries (INCI: Cetearyl Alcohol) | 5,00% |
| Preservative, Perfume | q.s. |

Formulierungsbeispiel 24) Rinse-Off Conditioner

| | |
|---|---|
| Water | 87,20% |
| VARISOFT® EQ 65, Evonik Industries (INCI: Distearoylethyl Dimonium Chloride; Cetearyl Alcohol) | 2,00% |
| VARISOFT® BT 85, Evonik Industries (INCI: Behentrimonium Chloride) | 2,00% |
| ABIL® Soft AF 100, Evonik Industries (INCI: Methoxy PEG/PPG-7/3 Aminopropyl Dimethicone) | 1,00% |
| Synthesebeispielen S8 | 2,80% |
| TEGO® Alkanol 1618, Evonik Industries (INCI: Cetearyl Alcohol) | 5,00% |
| Preservative, Perfume | q.s. |

Formulierungsbeispiel 25) Rinse-Off Conditioner

| | |
|---|---|
| Water | 88,20% |
| VARISOFT® BT 85, Evonik Industries (INCI: Behentrimonium Chloride) | 3,00% |
| SF 1708, Momentive (INCI: Amodimethicone) | 2,00% |
| Synthesebeispielen S6 | 1,80% |
| TEGO® Alkanol 1618, Evonik Industries (INCI: Cetearyl Alcohol) | 5,00% |
| Preservative, Perfume | q.s. |

Formulierungsbeispiel 26, feuchtigkeitsspendendes Hautreinigungsmittel

| | | |
|---|---|---|
| A | TEXAPON® NSO, BASF, 28%-ig (INCI: Sodium Laureth Sulfate) | 30,00% |
| | Synthesebeispielen S8 | 1,70% |
| | Perfume | 0,30% |

(fortgesetzt)

| | | |
|---|---|---|
| B | Water | 54,60% |
| | TEGOCEL® fluid HPM 4000, Evonik Industries (INCI: Hydroxypropyl Methylcellulose) | 1,20% |
| | TEGO® Betain C 60, Evonik Industries, 46%-ig (INCI: Cocamidopropyl Betaine) | 8,10% |
| | TEGOSOFT® APM, Evonik Industries (INCI: PPG-3 Myristyl Ether) | 1,00% |
| | Cutina TS, BASF (INCI: PEG- 3 Distearate) | 1,00% |
| | REWODERM® LI S 80, Evonik Industries (INCI: PEG-200 Hydrogenated Glyceryl Palmate; PEG-7 Glyceryl Cocoate) | 1,50% |
| | Preservative | 0,60% |
| | Citric Acid, 30%-ig | q.s. |

Formulierungsbeispiel 27, Duschgel

| | |
|---|---|
| TEXAPON® NSO, BASF, 28%-ig (INCI: Sodium Laureth Sulfate) | 15,00% |
| Synthesebeispielen S6 | 1,50% |
| Perfume | 0,30% |
| PGFAC-S, BASF (INCI: Sodium Cocoyl Hydrolyzed Wheat Protein Glutamate) | 1,50% |
| REWOPOL® SB CS 50 B, Evonik Industries, 40%-ig (INCI: Disodium PEG-5 Laurylcitrate Sulfosuccinate; Sodium Laureth Sulfate) | 7,50% |
| Water | 58,10% |
| TEGO® Betain F 50, Evonik Industries, 38%-ig (INCI: Cocamidopropyl Betaine) | 9,00% |
| TEGO® Betain 810, Evonik Industries, 38%-ig (INCI: Capryl/Capramidopropyl Betaine) | 4,00% |
| Polyquaternium-7, Nalco, (INCI: Merquat 550) | 0,50% |
| ANTIL® 200, Evonik Industries, (INCI: PEG-200 Hydrogenated Glyceryl Palmate; PEG-7 Glyceryl Cocoate) | 2,30% |
| Preservative | 0,30% |

Formulierungsbeispiel 28, Körperreinigungsmittel

| | | |
|---|---|---|
| A | TEXAPON® NSO BASF 28%-ig (INCI: Sodium Laureth Sulfate) | 30,00% |
| | Synthesebeispielen S8 | 1,50% |
| | ABIL® B 8832, Evonik Industries (INCI: Bis-PEG/PPG-20/20 Dimethicone) | 0,30% |
| | Perfume | 0,30% |
| B | Water | 51,00% |
| | TEGOCEL® fluid HPM 4000, Evonik Industries (INCI: Hydroxypropyl Methylcellulose) | 1,20% |
| | Citric Acid Monohydrate | 0,50% |
| | REWOTERIC® AM C, Evonik Industries, 32%-ig (INCI: Sodium Cocoamphoacetate) | 10,00% |
| | Cutina TS, BASF (INCI: PEG- 3 Distearate) | 2,00% |
| | REWODERM® LI S 80, Evonik Industries (INCI: PEG-200 Hydrogenated Glyceryl Palmate; PEG-7 Glyceryl Cocoate) | 2,60% |
| | Preservative | 0,60% |
| | Citric Acid, 30%-ig | q.s. |

Formulierungsbeispiel 29, Body Cleansing Foam

| | |
|---|---|
| TEXAPON® NSO, BASF, 28%-ig (INCI: Sodium Laureth Sulfate) | 14,00% |
| Perfume | 0,30% |
| Synthesebeispielen S6 | 0,70% |
| REWOTERIC® AM C, Evonik Industries, 32%-ig (INCI: Sodium Cocoamphoacetate) | 8,00% |
| Water | 74,80% |
| TEGOCEL® HPM 50, Evonik Industries (INCI: Hydroxypropyl Methylcellulose) | 0,50% |
| LACTIL®, Evonik Industries (INCI: Sodium Lactate; Sodium PCA; Glycine; Fructose; Urea; Niacinamide; Inositol; Sodium Benzoate; Lactic Acid) | 1,00% |
| Panthenol, BASF (INCI: D- Panthenol USP) | 0,20% |
| Citric Acid Monohydrate | 0,50% |

Formulierungsbeispiel 30, Turbid Conditioning Shampoo

| | |
|---|---|
| TEXAPON® NSO, BASF, 28%-ig (INCI: Sodium Laureth Sulfate) | 32,00% |
| ANTIL® 200, Evonik Industries (INCI: PEG-200 Hydrogenated Glyceryl Palmate; PEG-7 Glyceryl Cocoate) | 2,00% |
| Synthesebeispielen S8 | 1,00% |
| Perfume | 0,25% |
| Water | 53,25% |
| Polymer JR 400, Amerchol (INCI: Polyquaternium-10) | 0,20% |
| TEGO® Betain F 50, Evonik Industries, 38%-ig (INCI: Cocamidopropyl Betaine) | 8,00% |
| DC1503 Fluid, Dow Corning (INCI: Dimethicone; Dimethiconol) | 1,00% |
| TEGO® Pearl N 300 Evonik Industries (INCI: Glycol Distearate; Laureth-4; Cocamidopropyl Betaine) | 2,00% |
| NaCl | 0,30% |
| Preservative | q.s. |

Formulierungsbeispiel 31) Mild Hair & Body Wash, PEG- und Sulfate-free

| | |
|---|---|
| Plantacare® 1200 UP, BASF, 50%-ig (INCI: Lauryl Glucoside) | 11,40% |
| Plantacare® 818 UP, BASF, 51%-ig (INCI: Coco Glucoside) | 5,60% |
| Water | 61,60% |
| ANTIL® SOFT SC, Evonik Industries (INCI: Sorbitan Sesquicaprylate) | 0,90% |
| Synthesebeispielen S6 | 1,00% |
| TEGOSOFT® LSE 65 K SOFT, Evonik Industries (INCI: Sucrose Cocoate) | 1,50% |
| TEGO® Betain F 50, Evonik Industries, 38%-ig (INCI: Cocamidopropyl Betaine) | 18,00% |
| Perfume, preservative | q.s. |
| Citric Acid, 30% | q.s. |

Formulierungsbeispiel 32) Sprayable Hairmilk, PEG-free

| | | |
|---|---|---|
| A | Water | 95,30% |
| | Lactic Acid, 80%-ig | 0,40% |
| B | TEGO® AMID S 18, Evonik Industries (INCI: Stearamidopropyl Dimethylamine) | 1,20% |
| | TEGIN® G 1100 Pellets, Evonik Industries (INCI: Glycol Distearate) | 0,60% |
| | TEGO® Care PS, Evonik Industries (INCI: Methyl Glucose Sesquistearate) | 1,20% |
| | TEGOSOFT® DEC, Evonik Industries (INCI: Diethylhexyl Carbonate) | 0,30% |
| | Synthesebeispielen S6 | 1,00% |
| | Perfume, preservative | q.s. |

Formulierungsbeispiel 33: Pearlized Shampoo

| | |
|---|---|
| TEXAPON® NSO, Cognis, 28%-ig (INCI: Sodium Laureth Sulfate) | 32,00% |
| Synthesebeispielen S8 | 0,75% |
| Perfume | 0,25% |
| Water | 56,00% |
| TEGO® Betain F 50, Evonik Industries, 38%-ig (INCI: Cocamidopropyl Betaine) | 8,00% |
| TEGIN® D 1102, Evonik Industries (INCI: PEG-3 Distearate) | 1,00% |
| ANTIL® 171, Evonik Industries (INCI: PEG-18 Glyceryl Oleate/Cocoate) | 1,50% |
| NaCl | 0,50% |
| Preservative | q.s. |

Formulierungsbeispiel 34: "Two in One" Shampoo

| | | |
|---|---|---|
| A | TEXAPON® NSO, Cognis, 28%-ig (INCI: Sodium Laureth Sulfate) | 30,00% |
| | Perfume | 0,50% |
| | Stepanate® SCS, Stepan (INCI: Sodium Cumenesulfonate) | 1,00% |
| | Water | 16,25% |
| | TEGO® Betain F 50, Evonik Industries, 38%-ig (INCI: Cocamidopropyl Betaine) | 8,00% |
| B | TEXAPON® NSO, Cognis, 28%-ig (INCI: Sodium Laureth Sulfate) | 15,00% |
| | Stepanate® SCS, Stepan (INCI: Sodium Cumenesulfonate) | 1,00% |
| | Water | 5,00% |
| | REWOMID® C 212, Evonik Industries (INCI: Cocamide MEA) | 1,50% |
| | TEGIN® G 1100 Pellets, Evonik Industries, (INCI: Glycol Distearate) | 1,50% |
| C | TEXAPON® NSO, Cognis, 28%-ig (INCI: Sodium Laureth Sulfate) | 15,00% |
| | TEGO® Alkanol 16, Evonik Industries (INCI: Cetyl Alcohol) | 0,50% |
| | Synthesebeispielen S6 | 1,50% |
| | Dimethicone (10000 mPa·s) | 1,50% |
| | Stepanate® SCS, Stepan (INCI: Sodium Cumenesulfonate) | 1,00% |
| | Keltrol®, CP Telco (INCI: Xanthan Gum) | 0,75% |
| | Preservative | q.s. |

Formulierungsbeispiel 35: Conditioning Anti-Schuppen Shampoo

| | | |
|---|---|---|
| A | TEGIN® G 1100 Pellets, Evonik Industries (INCI: Glycol Distearate) | 3,00% |
| | TEXAPON® NSO, Cognis, 28%-ig (INCI: Sodium Laureth Sulfate) | 40,00% |
| B | Perfume | 0,30% |
| | Zinc-Pyrion NF, WeylChem, 48%-ig (INCI: Zinc Pyrithione) | 2,00% |
| | Synthesebeispielen S6 | 2,00% |
| C | Water | 35,70% |
| | TEGO® Carbomer 341 ER, Evonik Industries (INCI: Acrylates/C10-30 Alkyl Acrylate Crosspolymer) | 0,20% |
| | Water | 0,30% |
| | NaOH, 25%-ig | 0,30% |
| D | REWOTERIC® AM B U 185, Evonik Industries, 30%-ig (INCI: Undecylenamidopropyl Betaine) | 12,50% |
| | ANTIL® SPA 80, Evonik Industries (INCI: Isostearamide MIPA; Glyceryl Laurate) | 3,70% |
| E | Preservative | q.s. |

Formulierungsbeispiel 36: Haarfärbemittel

| | |
|---|---|
| Water demineralized | 57,40% |
| TEGO® Alkanol 1618, Evonik Industries, (INCI: Cetearyl Alcohol) | 12,00% |
| Eutanol® G, BASF (INCI: Octyldodecanol) | 3,00% |
| REWOMID® C 212, Evonik Industries (INCI: Cocamide MEA) | 1,50% |
| Super Hartolan® B, Crodo (INCI: Lanolin Alcohol) | 3,00% |
| Avocadoöl, Henry Lamotte (INCI: Persea Gratissima Oil) | 1,50% |
| Pristerene® 4960, Uniquema (INCI: Stearic Acid) | 6,00% |
| EDTA BD, BASF (INCI: Disodium EDTA) | 0,10% |
| Texapon® K12G, BASF (INCI: Sodium Lauryl Sulfate) | 0,50% |
| Propylenglycol | 5,00% |
| Timica Silver Sparkle, BASF (INCI: MICA; Titanium Dioxide) | 1,00% |
| Ammoniaklösung, 25%ig | 6,00% |
| 2,5-Diaminotoluolsulfat, (INCI: Toluene-2,5-Diamine) | 1,40% |
| Rodol® RS, Jos. H. Lowenstein & Sons (INCI: Resorcinol) | 0,30% |
| HC Blue A42, (INCI: 2,4-Diaminophenoxyethanol di HCl) | 0,10% |
| Natriumsulfit | 0,50% |
| Perfume | 0,20% |
| Synthesebeispielen S8 | 0,50% |

Formulierungsbeispiel 37: Haarfärbemittel

| | |
|---|---|
| Water demineralized | 64,00% |
| TEGO® Alkanol 1618, Evonik Industries (INCI: Cetearyl Alcohol) | 12,00% |

(fortgesetzt)

| | |
|---|---|
| Super Hartolan® B, Croda (INCI: Lanolin Alcohol) | 2,50% |
| Meadowfoam® Seed Oil, Fanning (INCI: Limnanthes Alba) | 1,00% |
| Pristerene® 4960, Uniquema (INCI: Stearic Acid) | 5,50% |
| EDTA BD, BASF (INCI: Disodium EDTA) | 0,10% |
| Glycerin | 5,00% |
| Texapon® N 70, BASF (INCI: Sodium Laureth Sulfate) | 2,00% |
| Monoethanolamin | 4,00% |
| 2,5-Diaminotoluolsulfat, (INCI: Toluene-2,5-Diamine) | 0,90% |
| Rodol® RS, Jos. H. Lowenstein & Sons (INCI: Resorcinol) | 0,20% |
| Jarocol® 4A3MP, Vivimed Labs (INCI: 4-Amino-M-Cresol) | 0,60% |
| Rodol® PAOC, Jos. H. Lowenstein & Sons (INCI: 4-Amino-2-Hydroxytoluene) | 0,50% |
| Uantox® EBATE, Universal Preserv-A-Chem (INCI: Erythorbic Acid) | 0,50% |
| Perfume | 0,20% |
| Synthesebeispielen S6 | 1,00% |

Formulierungsbeispiel 38: Haarfärbemittel

| | |
|---|---|
| Water demineralized | 67,50% |
| TEGO® Alkanol 1618, Evonik Industries (INCI: Cetearyl Alcohol) | 10,00% |
| Eutanol® G, BASF Cognis (INCI: Octyldodecanol) | 1,00% |
| REWOMID® C 212, Evonik Industries (INCI: Cocamide MEA) | 2,00% |
| TEGIN® VS, Evonik Industries (INCI: Glyceryl Stearate SE) | 5,00% |
| Fitoderm®, Hispano Quimica S. A. (INCI: Squalane) | 1,00% |
| Coenzyme Q 10 | 0,10% |
| EDTA BD, BASF (INCI: Disodium EDTA) | 0,10% |
| Texapon® K12G, BASF (INCI: Sodium Lauryl Sulfate) | 0,10% |
| Propylenglycol | 5,00% |
| Ammoniaklösung, 25%ig | 3,00% |
| Rodol® ERN, Jos. H. Lowenstein & Sons (INCI: 1-Naphthol) | 0,30% |
| Imexine® OAG, Chimex (INCI: 2-Methyl-5-Hydroxyethylaminophenol) | 1,00% |
| Colorex® WP5, Teluca (INCI: 1-Hydroxyethyl 4,5-Diamino Pyrazole Sulfate) | 2,60% |
| Ascorbinsäure | 0,30% |
| Perfume | 0,30% |
| Synthesebeispielen S8 | 0,70% |

Formulierungsbeispiel 39: Haarfärbemittel

| | |
|---|---|
| Water demineralized | 60,70% |
| TEGO® Alkanol 1618, Evonik Industries (INCI: Cetearyl Alcohol) | 13,00% |
| REWOMID® C 212, Evonik Industries (INCI: Cocamide MEA) | 2,00% |

(fortgesetzt)

| | |
|---|---|
| Super Hartolan® B, Croda (INCI: Lanolin Alcohol) | 2,50% |
| Avocadoöl, Henry Lamotte (INCI: Persea Gratissima Oil) | 2,00% |
| Pristerene® 4960, Uniquema (INCI: Stearic Acid) | 6,00% |
| EDTA BD, BASF (INCI: Disodium EDTA) | 0,10% |
| Texapon® K12G, BASF (INCI: Sodium Lauryl Sulfate) | 0,10% |
| Propylenglycol | 5,00% |
| Ammoniaklösung, 25%ig | 6,00% |
| 2,5-Diaminotoluolsulfat, (INCI: Toluene-2,5-Diamine) | 1,30% |
| Rodol® RS, Jos. H. Lowenstein & Sons (INCI: Resorcinol) | 0,30% |
| Covastyle® TBQ, LCW Les colorants Wackherr S.A. (INCI: t-Butyl Hydroquinone) | 0,30% |
| Perfume | 0,20% |
| Synthesebeispielen S6 | 0,50% |

Formulierungsbeispiel 40: Shampoo

| | |
|---|---|
| TEXAPON® NSO, Cognis, 28%-ig (INCI: Sodium Laureth Sulfate) | 28,00% |
| REWOTERIC® AM 2 C NM, Evonik Industries, 39%-ig (INCI: Disodium Cocoamphodiacetate) | 4,00% |
| TEGO® Betain F 50, Evonik Industries, 38%-ig (INCI: Cocamidopropyl Betaine) | 7,00% |
| REWOMID® C 212, Evonik Industries (INCI: Cocamide MEA) | 0,80% |
| ANTIL® 171, Evonik Industries (INCI: PEG-18 Glyceryl Oleate/Cocoate) | 0,50% |
| N-Hance® SP-100, Hercules (INCI: Acrylamidopropyl Trimonium Chloride/Acrylamide Copolymer) | |
| Polymer JR 400, Amerchol (INCI: Polyquaternium-10) | 0,10% |
| Jaguar C-162, Rhodia (INCI: Hydroxypropyl Guar Hydroxypropyltrimonium Chloride) | 0,20% |
| DC 193, Dow Corning (INCI: PEG-12 Dimethicone) | 0,40% |
| Synthesebeispielen S8 | 0,70% |
| Synthesebeispielen S5 | 0,60% |
| TEGIN® D 1102, Evonik Industries (INCI: PEG-3 Distearate) | 0,40% |
| TAGAT® CH 40, Evonik Industries (INCI: PEG-40 Hydrogenated Castor Oil) | 0,20% |
| Water | 56,00% |
| NaCl | 0,70% |
| Citric Acid | ad. pH=-5,5 |
| Perfume | q.s. |
| Preservative | q.s. |

**Patentansprüche**

**1.** Verfahren zu Herstellung von aminosäuremodifizierten Siloxanen umfassend die Verfahrensschritte

C) Umsetzen eines Salzes der allgemeinen Formel $K^+A^-$ mit mindestens einem mindestens eine Epoxy- und/oder Carbonatgruppe aufweisenden Siloxan, wobei
$K^+$ ein organisches Kation und

$A^-$ das Carboxylat einer α-Amino-Carbonsäure, gegebenenfalls D) Umsetzen mit einer weiteren epoxygruppenhaltigen, organischen Komponente und gegebenenfalls E) Aufreinigen des aminosäuremodifizierten Siloxans.

**2.** Verfahren gemäß Anspruch 1, **dadurch gekennzeichnet, dass** als organisches Kation $K^+$ Verbindungen enthaltend eine Ammonium-Gruppe oder eine Phosphonium-Gruppe eingesetzt werden.

**3.** Verfahren gemäß Anspruch 1 oder 2, **dadurch gekennzeichnet, dass** als organisches Kation $K^+$ eine Verbindung der allgemeinen Formel I

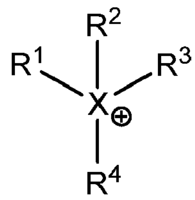

allgemeine Formel I

mit X = Stickstoff oder Phosphor und
$R^1$, $R^2$, $R^3$, $R^4$ gleich oder unterschiedlich, linearer oder verzweigter aliphatischer Kohlenwasserstoffrest mit 1 bis 30 Kohlenstoffatomen, mit der Maßgabe, dass mindestens einer der Reste $R^1$, $R^2$, $R^3$, $R^4$ 4 bis 30 Kohlenstoffatome aufweist,
eingesetzt wird.

**4.** Verfahren gemäß mindestens einem der vorherigen Ansprüche, **dadurch gekennzeichnet, dass** als A- das Carboxylat einer der 22 proteinogenen Aminosäuren, welche gegebenenfalls glykosyliert sein können, insbesondere ausgewählt aus Lysin, Arginin und Histidin,
eingesetzt wird.

**5.** Verfahren gemäß mindestens einem der vorherigen Ansprüche, **dadurch gekennzeichnet, dass** ein Siloxan der allgemeinen Formel IV eingesetzt wird

$$M_{a1}\ M^A_{a2} M^B_{a3} D_{b1}\ D^A_{b2} D^B_{b3} T_{c1} T^A_{c2} T^B_{c3} Q_{d1} \qquad \text{allgemeine Formel IV}$$

mit

$M = [R^{16}{}_3SiO_{1/2}]$
$M^A = [R^{17}R^{16}{}_2SiO_{1/2}]$
$M^B = [R^{18}R^{16}{}_2SiO_{1/2}]$
$D = [R^{16}{}_2SiO_{2/2}]$
$D^A = [R^{17}{}_1R^{16}{}_1SiO_{2/2}]$
$D^B = [R^{18}{}_1R^{16}{}_1SiO_{2/2}]$
$T = [R^{16}SiO_{3/2}]$
$T^A = [R^{17}SiO_{3/2}]$
$T^B = [R^{1B}SiO_{3/2}]$
$Q = [SiO_{4/2}]$,

wobei gilt

$R^{16}$ unabhängig voneinander gleiche oder verschiedene lineare oder verzweigte, gesättigte oder ungesättigte Kohlenwasserstoffreste mit 1 bis 30 Kohlenstoffatomen oder auch aromatische Kohlenwasserstoffreste mit 6 bis 30 Kohlenstoffatomen bevorzugt Methyl oder Phenyl, insbesondere Methyl,
$R^{17}$ unabhängig voneinander gleiche oder verschiedene epoxy- und/oder carbonatgruppenhaltige Reste,
$R^{18}$ unabhängig voneinander gleiche oder verschiedene lineare oder verzweigte, gesättigte oder olefinisch ungesättigte Kohlenwasserstoffreste mit 8 bis 30 Kohlenstoffatomen, beispielsweise Decyl-, Dodecyl, Tetradecyl-, Hexadecyl-, Octadecyl-, insbesondere Hexadecyl- und Octadecyl-,

einen aromatischen Kohlenwasserstoffrest mit 6 bis 40 Kohlenstoffatomen, einen Alkylarylrest mit 7 bis 40 Kohlenstoffatomen,
einen durch ein oder mehrere Heteroatome (Sauerstoff, NH, NR' mit R' gleich einem gegebenenfalls Doppelbindungen enthaltenden C1 bis C30-Alkylrest, insbesondere $-CH_3$) unterbrochenen linearen oder verzweigten gegebenenfalls Doppelbindungen enthaltenden aliphatischen Kohlenwasserstoffrest mit 2 bis 30 Kohlenstoffatomen,
einen durch eine oder mehrere Funktionalitäten, ausgewählt aus der Gruppe -O-C(O)-, -(O)C-O-, -NH-C(O)-, -(O)C-NH, - $(CH_3)$N-C (O)-, -(O)C-N$(CH_3)$-, - S$(O_2)$-O-, -O-S$(O_2)$-, -S$(O_2)$-NH-, -NH-S$(O_2)$-, -S $(O_2)$-N $(CH_3)$-, -N$(CH_3)$-S$(O_2)$-, unterbrochenen linearen oder verzweigten gegebenenfalls Doppelbindungen enthaltenden aliphatischen Kohlenwasserstoffrest mit 2 bis 30 Kohlenstoffatomen,
einen endständig OH, OR', $NH_2$, N(H)R', $N(R')_2$ (mit R' gleich einem gegebenenfalls Doppelbindungen enthaltenden C1- bis C30-Alkylrest) funktionalisierten linearen oder verzweigten gegebenenfalls Doppelbindungen enthaltenden aliphatischen oder cycloaliphatischen Kohlenwasserstoffrest mit 1 bis 30 Kohlenstoffatomen oder
einen blockweise oder statistisch aufgebauten Polyether gemäß $-(R^5-O)_n-R^6$, wobei $R^5$ ein 2 bis 4 Kohlenstoffatome enthaltender linearer oder verzweigter Kohlenwasserstoffrest, n 1 bis 100, vorzugsweise 2 bis 60, ist und $R^6$ Wasserstoff, einen linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einen aromatischen Kohlenwasserstoffrest mit 6 bis 40 Kohlenstoffatomen, einen Alkylarylrest mit 7 bis 40 Kohlenstoffatomen,
oder ein Rest $-C(O)-R^7$ mit $R^7$ gleich einem linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einem aromatischen Kohlenwasserstoffrest mit 6 bis 40 Kohlenstoffatomen, einem Alkylarylrest mit 7 bis 40 Kohlenstoffatomen,

a1 = 0 bis 200, bevorzugt 1 bis 60, insbesondere 0,
a2 = 0 bis 30, bevorzugt 1 bis 20, insbesondere 2 bis 10,
a3 = 0 bis 30, bevorzugt 1 bis 20, insbesondere 0,
b1 = 3 bis 5000, bevorzugt 3 bis 1000, insbesondere 10 bis 500,
b2 = 0 bis 100, bevorzugt 1 bis 30, insbesondere 1 bis 10,
b3 = 0 bis 100, bevorzugt 0 bis 30, insbesondere 0,
c1 = 0 bis 30, bevorzugt 1 bis 30, alternativ bevorzugt 0,
c2 = 0 bis 30, bevorzugt 0 bis 10, insbesondere 0 bis 5,
c3 = 0 bis 30, bevorzugt 0 bis 5, insbesondere 0,
d1 = 0 bis 30, bevorzugt 1 bis 5, alternativ bevorzugt 0,

mit der Maßgabe, dass mindestens einer der Indices a2, b2 oder c2 # 0 ist.

**6.** Verfahren gemäß mindestens einem der vorherigen Ansprüche, **dadurch gekennzeichnet, dass** es die zusätzlichen Verfahrensschritte umfasst

A) Umsetzen einer $\alpha$-Amino-Carbonsäure $H^+A^-$ mit einem organischen Salz des organischen Kations $K^+$ in Anwesenheit einer Base zu einem Salz der allgemeinen Formel $K^+A^-$,
und gegebenenfalls
B) Aufreinigen des Salzes der allgemeinen Formel $K^+A^-$.

**7.** Verfahren gemäß Anspruch 6, **dadurch gekennzeichnet, dass** das Gegenanion des organischen Salzes des organischen Kations $K^+$ ausgewählt ist aus der Gruppe Halogenide, Hydroxide, Bis(perfluoralkylsulfonyl)amide, Alkyl- und Aryltosylate, Perfluoralkyltosylate, Nitrate, Sulfate, Hydrogensulfate, Alkyl- und Arylsulfate, Polyethersulfate und -sulfonate, Perfluoralkylsulfate, Sulfonate, Alkyl- und Arylsulfonate, perfluorierte Alkyl- und Arylsulfonate, Alkyl- und Arylcarboxylate, Perfluoralkylcarboxylate, Perchlorate, Tetrarchloroaluminate, Saccharinate, Dicyanamid, Tetrafluoroborat, Hexafluorophosphat, Polyether-Phosphate und Phosphat.

**8.** Verfahren gemäß Anspruch 6 oder 7 **dadurch gekennzeichnet, dass** als Base Verbindungen eingesetzt werden ausgewählt aus der Gruppe Alkali- und Erdalkalimetallhydroxide wie Natriumhydroxid, Kaliumhydroxid, Calciumhydroxid, Bariumhydroxid, Alkoholate der Alkali- oder Erdalkalimetalle und Ammonium- und Phosphoniumhydroxide.

**9.** Aminosäuremodifiziertes Siloxan erhältlich nach einem Verfahren gemäß mindestens einem der vorherigen Ansprüche.

**10.** Siloxan der allgemeinen Formel VI

$$M_{a1}\ M^{C}_{a2}M^{B}_{a3}D_{b1}\ D^{C}_{b2}D^{B}_{b3}T_{c1}T^{C}_{c2}T^{B}_{c3}Q_{d1} \qquad \text{allgemeine Formel VI}$$

mit

$M = [R^{16}{}_3SiO_{1/2}]$
$M^C = [R^{19}R^{16}{}_2SiO_{1/2}]$
$M^B = [R^{18}R^{16}{}_2SiO_{1/2}]$
$D = [R^{16}{}_2SiO_{2/2}]$
$D^C = [R^{19}{}_1R^{16}{}_1SiO_{2/2}]$
$D^B = [R^{18}{}_1R^{16}{}_1SiO_{2/2}]$
$T = [R^{16}SiO_{3/2}]$
$T^C = [R^{19}SiO_{3/2}]$
$T^B = [R^{18}SiO_{3/2}]$
$Q = [SiO_{4/2}]$,

wobei gilt

a1, a2, a3, b1, b2, b3, c1, c2, c3, d1, $R^{16}$ und $R^{18}$ wie in Anspruch 5 definiert,
$R^{19}$ unabhängig voneinander gleiche oder verschiedene Reste der allgemeinen Formel VIIa und/oder VIIb

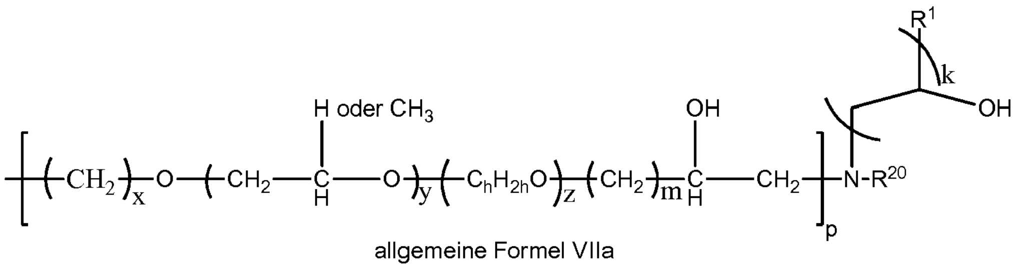

allgemeine Formel VIIa

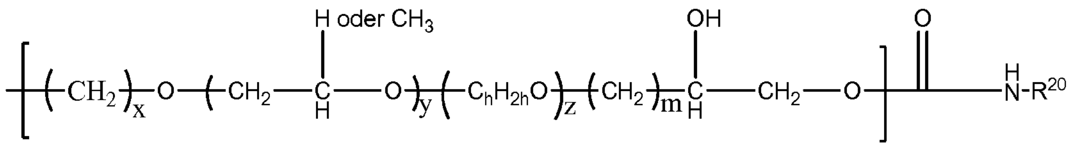

allgemeine Formel VIIb

mit p = 1-2,
k = 0-1,
m = 1-20, x = 1-20, y = 0-10, z = 0-50 und h = 1-10,
$R^1$ wie in Anspruch 3 definiert,

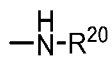

sich ableitet von in Anspruch 1 definiertem $K^+A^-$, welches unter Verlust eines H an einer Aminogruppe mit dem Rest $R^{19}$ gebunden ist und

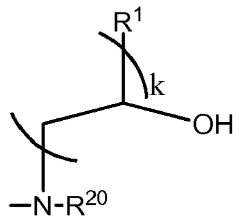

sich ableitet von

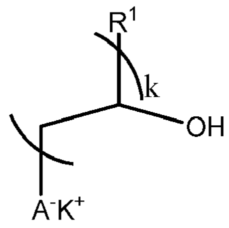

mit $K^+A^-$, wie in Anspruch 1 definiert, welches unter Verlust eines H an einer Aminogruppe mit dem Rest $R^{19}$ gebunden ist.

**11.** Salz der allgemeinen Formel $K^+A^-$ mit
$K^+$ ein organisches Kation und
$A^-$ das Carboxylat einer α-Amino-Carbonsäure, **dadurch gekennzeichnet, dass**
$K^+$ eine Verbindung enthaltend eine Ammonium-Gruppe darstellt, die mindestens einen organischen Rest mit 8 bis 30 Kohlenstoffatomen aufweist.

**12.** Salz gemäß Anspruch 11, **dadurch gekennzeichnet, dass** das organisches Kation $K^+$ eine Verbindungen der allgemeinen Formel I

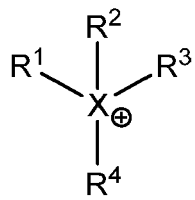

allgemeine Formel I

mit X = Stickstoff und
$R^1$, $R^2$, $R^3$, $R^4$ gleich oder unterschiedlich sind und einen linearen oder verzweigten aliphatischen Kohlenwasserstoffrest mit 1 bis 30 Kohlenstoffatomen bedeuten, mit der Maßgabe, dass mindestens einer der Reste $R^1$, $R^2$, $R^3$, $R^4$ 4 bis 30 Kohlenstoffatome, aufweist,

**13.** Formulierung enthaltend mindestens ein Siloxan gemäß Anspruch 9 oder 10.

**14.** Verwendung mindestens eines Siloxans gemäß Anspruch 9 oder 10 oder einer Formulierung gemäß Anspruch 13 zur Konditionierung einer Oberfläche, bevorzugt von Fasern oder Geweben, insbesondere von Haut, Haaren oder Textilien.

Europäisches Patentamt
European Patent Office
Office européen des brevets

# EUROPÄISCHER RECHERCHENBERICHT

Nummer der Anmeldung
EP 14 17 2890

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | YONGSHENG WANG ET AL: "The self-organization properties of n-dodecylammonium [alpha]-glutamate/n-C5H11OH/water system", COLLOID AND POLYMER SCIENCE ; KOLLOID-ZEITSCHRIFT UND ZEITSCHRIFT FÜR POLYMERE, SPRINGER, BERLIN, DE, Bd. 285, Nr. 13, 13. Juli 2007 (2007-07-13), Seiten 1423-1431, XP019541445, ISSN: 1435-1536, DOI: 10.1007/S00396-007-1698-5 * "GDA"; Seite 1423, Spalte 2 * ----- | 11 | INV. C08G77/388 C07C227/18 C07C279/14 C07C211/63 C07C229/26 |
| X | RODEHÜSER L ET AL: "Derivatives of glutamic acid as new surfactants", AMINO ACIDS, SPRINGER VERLAG, AU, Bd. 18, Nr. 1, 1. Januar 2000 (2000-01-01), Seiten 89-100, XP002334167, ISSN: 0939-4451, DOI: 10.1007/S007260050008 * Scheme 2 * ----- | 11 | RECHERCHIERTE SACHGEBIETE (IPC): C08G C07C |
| X | RAMESH L. GARDAS ET AL: "Thermophysical Properties of Amino Acid-Based Ionic Liquids", JOURNAL OF CHEMICAL & ENGINEERING DATA, Bd. 55, Nr. 4, 8. April 2010 (2010-04-08), Seiten 1505-1515, XP055136638, ISSN: 0021-9568, DOI: 10.1021/je900660x * Tabelle 1 * ----- | 11,12 | |
| A,D | WO 2009/084711 A1 (DOW CORNING TORAY CO LTD [JP]; DOW CORNING [US]; DOW CORNING EUROP SA) 9. Juli 2009 (2009-07-09) * Seiten 13-15; Beispiel 1 * ----- | 1-10,13, 14 | |

1

Der vorliegende Recherchenbericht wurde für alle Patentansprüche erstellt

| Recherchenort | Abschlußdatum der Recherche | Prüfer |
|---|---|---|
| Den Haag | 27. August 2014 | Dalet, Pierre |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 14 17 2890

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-08-2014

| Im Recherchenbericht angeführtes Patentdokument | Datum der Veröffentlichung | Mitglied(er) der Patentfamilie | Datum der Veröffentlichung |
|---|---|---|---|
| WO 2009084711 A1 | 09-07-2009 | CN 101918475 A | 15-12-2010 |
| | | EP 2231753 A1 | 29-09-2010 |
| | | JP 5383035 B2 | 08-01-2014 |
| | | JP 2009155564 A | 16-07-2009 |
| | | KR 20100106422 A | 01-10-2010 |
| | | US 2010310496 A1 | 09-12-2010 |
| | | WO 2009084711 A1 | 09-07-2009 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1149855 A
- JP 2004182680 A
- US 5516869 A
- US 5412074 A
- EP 1477512 A1
- WO 2009084711 A1
- US 5606077 A
- DE 10327871